(19) Europäisches Patentamt
European Patent Office
Office européen des brevets

(11) Publication number: **0 038 195**
**B1**

# (12) EUROPEAN PATENT SPECIFICATION

(45) Date of publication of patent specification: **03.10.84**

(51) Int. Cl.$^3$: **C 07 H 15/20,** A 61 K 31/70

(21) Application number: **81301593.0**

(22) Date of filing: **10.04.81**

## (54) Phenylamino saccharide derivatives, their production and pharmaceutical compositions containing them.

(30) Priority: **11.04.80 JP 47654/80**

(43) Date of publication of application:
**21.10.81 Bulletin 81/42**

(45) Publication of the grant of the patent:
**03.10.84 Bulletin 84/40**

(84) Designated Contracting States:
**BE CH DE FR GB IT LI SE**

(56) References cited:
**GB-A-2 018 135**
**GB-A-2 018 592**
**GB-A-2 022 411**
**GB-A-2 029 698**

**Annual Reports in Medicinal Chemistry Vol. 10 (1975) pages 306-14**

**The file contains technical information submitted after the application was filed and not included in this specification**

(73) Proprietor: **KUREHA KAGAKU KOGYO KABUSHIKI KAISHA**
**9-11 *Horidome-cho 1-chome Nihonbashi Chuo-ku***
**Tokyo (JP)**

(72) Inventor: **Yoshikumi, Chikao**
**2-19-46 Higashi Kunitachi-shi**
**Tokyo (JP)**
Inventor: **Hirose, Fumio**
**2-3-9 Zenpukuji Suginami-ku**
**Tokyo (JP)**
Inventor: **Ohmura, Yoshio**
**5-48-3 Maebara-Higashi**
**Funabashi-shi Chiba-ken (JP)**
Inventor: **Fujii, Takayoshi**
**5-11-21 Towa Adachi-ku**
**Tokyo (JP)**
Inventor: **Ikuzawa, Masanori**
**1-25-20 Wakaba-cho Tachikawa-shi**
**Tokyo (JP)**
Inventor: **Ohhara, Minoru**
**19-25 Fujimi-cho Itabashi-ku**
**Tokyo (JP)**
Inventor: **Matsunaga, Kenichi**
**3-26-1 Hyakunin-cho**
**Shinjuku-ku Tokyo (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

(72) Inventor: **Ando, Takao**
**5-31-1 Matsubara Setagaya-ku**
**Tokyo (JP)**

(74) Representative: **Myerscough, Philip Boyd et al**
**J.A.Kemp & Co. 14, South Square Gray's Inn**
**London, WC1R 5EU (GB)**

**Description**

The present invention relates to saccharide derivatives, to their preparation and to pharmaceutical compositions containing them.

GB—A—2022411, GB—A—2029698 and GB—A—2018592 disclose compounds represented by the formula:

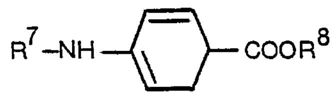

wherein $R^7$ denotes a group formed by the removal of OH from the 1-(alpha) or 1-(beta) position of arabinose, glucose, galactose, mannose, xylose or rhamnose, and $R^8$ denotes H, Na, K, 1/2 Mg, 1/2 Ca or 1/3 Al. GB—A—2018135 A discloses compounds represented by the formula:

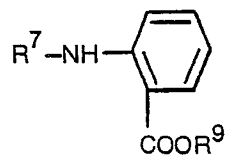

wherein $R^7$ denotes a group formed by the removal of OH from the 1-(alpha) or 1-(beta) position of arabinose, xylose, glucose, galactose, rhamnose or mannose, and $R^9$ denotes H, Na, K, 1/2 Mg, 1/2 Ca, 1/3 Al or methyl.

The present invention provides saccharide derivatives represented by the general formula (I):

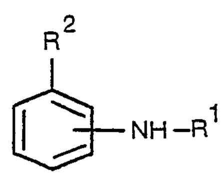

wherein $R^1$ represents the residue formed by removing the hemiacetal hydroxy group from a mono-, di- or trisaccharide, an aminosaccharide, D-glucuronic acid or L-gulonic acid, $R^2$ represents

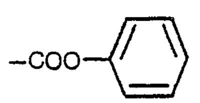 , 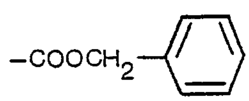 , 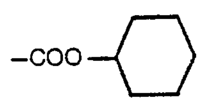 ,

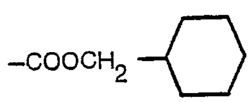 , $-CONH_2$ or $-CH_2COOR^3$

and $R^3$ represents a hydrogen atom, a lower alkyl group or one equivalent of a pharmaceutically acceptable metal. The lower alkyl group can have from 1 to 6, preferably from 1 to 4 carbon atoms.

These compounds can be prepared according to the invention by reacting a compound of formula [II]

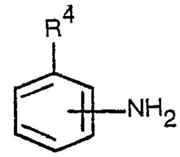

[II]

wherein $R^4$ represents 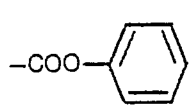 , 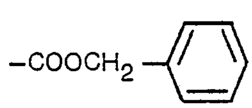 , 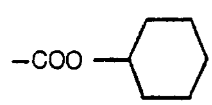 ,

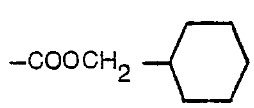 , $-CONH_2$ or $-CH_2COOR^5$

and $R^5$ represents a hydrogen atom or a lower alkyl group, with a mono-, di- or tri-saccharide, an aminosaccharide, D-glucuronic acid or L-gulonic acid, and, if desired, converting a resulting compound

wherein $R^4$ represents $-CH_2COOH$ to a compound wherein $R^4$ represents $-CH_2COOR^6$, $R^6$ representing an equivalent of a pharmaceutically acceptable metal.

The derivatives of the invention are useful as pharmaceutical agents because of their physiological activity in reducing blood sugar levels and reducing blood pressure. They also possess anti-tumour, suppressing central nerve and anti-inflammatory activity and have an extremely low acute mammalian toxicity without exhibiting any side effects even after prolonged administration. They can therefore be employed as the active ingredient in pharmaceutical compositions with a pharmaceutically acceptable carrier or diluent.

In the description which follows, reference will be made to the accompanying drawings in which Figures 1 to 29 show the respective infrared absorption spectra of compounds of the present invention.

The group $R^1$ can represent the residue formed by removing the hemiacetal hydroxyl group from a mono-, di- or trisaccharide. This can be the residue formed by removing a hydroxyl group from the reducing end of these saccharides. The mono-, di- or trisaccharide may be a D-isomer, L-isomer, alpha-anomer, beta-anomer or a mixture of these anomers. Examples of the mono-, di- and tri-saccharides include pentoses such as D-ribose, D-xylose, D- or L-arabinose, D-lyxose, L- or D-xylulose and D-ribulose, hexoses such as D- or L-galactose, D-glucose, D-mannose, D-fructose, L-sorbose, D-tagatose and D-psicose, heptoses such as D-mannoheptulose and D-sedoheptulose, disaccharides such as maltose, sucrose, cellobiose, lactose, laminaribiose, gentiobiose, melibiose, isomaltose, mannobiose and xylobiose, trisaccharides such as mannotriose, xylotriose, gentiotriose and maltotriose, and deoxysaccharides such as 2-deoxy-D-ribose, 6-deoxygalactose, 6-deoxymannose and digitoxose.

Moreover, in addition to the abovementioned saccharides, $R^1$ of the general formula (I) includes also the residues of aminosaccharides such as glucosamine, galactosamine, N-acetyl-D-glucosamine, and D-N-acetyl-muramic acid; D-glucuronic acid; and L-gulonic acid.

Preferably, $R^1$ is the group formed by removing the hemiacetal hydroxy group from ribose, deoxyribose, glucose, fructose, xylose, mannose, rhamnose, fucose, lactose, cellobiose or maltotriose.

In the case where $R^2$ of the general formula (I) represents $-CH_2COOR^3$ and $R^3$ represents a lower alkyl group or an equivalent of a pharmaceutically acceptable metal, $R^3$ can represent an alkali metal such as sodium and potassium, an alkaline earth metal such as calcium and magnesium, aluminum, and an alkyl group such as methyl, ethyl, propyl and butyl.

A saccharide derivative according to the present invention (hereinafter referred to as the present substance) can be produced by the following method:

In $2 \times 10^{-6}$ to $2 \times 10^{-4} m^3$ (2 to 200 ml) of a solvent, for example water, methanol, ethanol, acetone, chloroform, dioxan or dimethylformamide, $10^{-3}$ to $10^{-2}$ kg (1 to 10 g) of saccharide is brought into reaction with $10^{-3}$ to $10^{-2}$ kg (1 to 10 g) of an ester of an aminobenzoic acid, an aminophenylacetic acid, an ester thereof or an aminobenzoic acid amide in the presence or absence of a catalyst at 20 to 200°C, preferably at 50 to 150°C for 10 min to 48 hours, preferably for 30 min to 24 hours. The thus obtained reaction mixture is cooled, and can subsequently be condensed to collect the crystals deposited. The crystals collected are washed with water, methanol, acetone or ether and then recrystallized. In cases where the present substance cannot be collected as crystals from the abovementioned reaction mixture, the reaction mixture is isolated, for example by thin layer chromatography or column chromatography, to collect the present substance as a syrup or powder.

The catalyst which can be used in the production of the present substance is preferably acetic acid, a salt thereof, hydrochloric acid or ammonium chloride. The catalyst is used in an amount of 0.1 to 5 kg per 1 to 10 kg of the saccharide. Where the saccharide is a disaccharide or trisaccharide, the use of ammonium chloride as the catalyst is not always favorable, and acetic acid is preferably used.

Where a disaccharide or trisaccharide is used as the starting material, the use of $10^{-6}$ to $5 \times 10^{-6} m^3$ (1 to 5 ml) of acetic acid per $2 \times 10^{-3}$ to $6 \times 10^{-3}$ kg (2 to 6 g) of the aminobenzoic acid amide, ester of aminobenzoic acid (including the phenyl-, benzyl-, cyclohexyl- and hexahydrobenzyl esters), or aminophenylacetic acid or lower alkyl ester thereof in $2 \times 10^{-6}$ to $2 \times 10^{-4} m^3$ (2 to 200 ml) of a solvent gives a favorable result. Use of less acetic acid causes a reduction in the yield of the present substance. On the other hand, use of acetic acid does not give an improved yield.

Where the present substance to be prepared is represented by the general formula (I) wherein $R^3$ is represented by a group $-CH_2COOM$ in which M is an equivalent of a metal, it is better to prepare that substance by replacing the hydrogen atom of the group $-CH_2COOH$ of the substance in which $R^3$ is $-CH_2COOH$ by well known methods.

Some of the present substances and their physiocochemical properties are exemplified in Table 1. As has been stated, their respective infrared absorption spectra are shown in Figs. 1 to 29 of the accompanying drawings. The number of the substances corresponds to the number of the figures and to the number of the Examples. For example, Fig. 1 shows the infrared absorption spectrum of substance No. 1 prepared in Example 1.

The toxicological properties of the present substances are shown as follows:

1) Acute toxicity to mammals:

The oral toxicity of each of the substances of Examples 1 to 29 was examined by administering orally each of them as a solution in distilled water or as a suspension in distilled water forcibly via a

stomach tube to ICR—JCL mice and observing the mice's mortality 7 days after the administration. An $LD_{50}$ value for acute oral toxicity was obtained by the Litchfield-Wilcoxon method. The values obtained for the substances produced in Examples 1 to 29, namely Compounds No. 1 to No. 29, are shown in Table 2. As seen in Table 2, each of the twenty nine compounds gives a large value of $LD_{50}$ showing that their acute oral toxicities are extremely low.

TABLE 1

Physicochemical properties of the present substances

| No. of Substance | Name of substance | Melting point (°C) | Specific rotatory power (°)* | | Elementary analytical composition (%) C | H | N | Ultra violet absorption max.** |
|---|---|---|---|---|---|---|---|---|
| 1 | o-Aminobenzoic acid amide-N-D-fructoside | 150—159 | −16.0 | C=0.5 methanol | 53.0 (53.1 | 6.0 6.1 | 8.3 8.2)*** | 225 and 250 |
| 2 | m-Aminobenzoic acid amide-N-cellobioside | 211—213 (decomp.) | −39.0 | C=0.05 ethanol | 48.1 (48.3 | 6.0 5.9 | 5.7 5.9) | 225, 245 and 310 |
| 3 | p-Aminobenzoic acid amide-N-D-xyloside | 184—186 (decomp.) | −32.0 | C=0.2 methanol | 53.7 (53.7 | 6.1 6.0 | 10.4 10.4) | 285 |
| 4 | p-Aminobenzoic acid amide-N-D-mannoside | 195—197 (decomp.) | ·110.0 | C=0.1 ethanol | 53.0 (53.1 | 6.1 6.1 | 8.1 8.2) | 285 |
| 5 | p-Aminobenzoic acid amide-N-L-rhamnoside | 164—170 (decomp.) | −54.6 | C=0.5 ethanol | 55.2 (55.3 | 6.4 6.4 | 9.8 9.9) | 285 |
| 6 | Phenyl o-aminobenzoate-N-L-rhamnoside | 201—202 (decomp.) | −32.4 | C=0.5 methanol | 63.4 (63.5 | 5.9 5.8 | 4.0 3.9) | 223, 252 and 343 |
| 7 | Phenyl m-aminobenzoate-N-D-mannoside | 183—185 (decomp.) | −104 | C=0.1 ethanol | 60.8 (60.8 | 5.6 5.6 | 3.8 3.7) | 223, 245 and 320 |
| 8 | Phenyl p-aminobenzoate-N-D-xyloside | 77—72 | +4.4 | C=0.5 methanol | 60.7 (60.8 | 8.1 8.2 | 4.0 3.9) | 223, 296 |
| 9 | Phenyl p-aminobenzoate-N-cellobioside | 140—144 | −42.5 | C=0.5 methanol | 61.3 (61.3 | 6.5 6.3 | 2.9 2.9) | 223, 296 |
| 10 | Phenyl p-aminobenzoate-N-maltotrioside | 155—170 | +73.2 | C=0.5 methanol | 43.5 (43.5 | 7.0 7.1 | 2.5 2.4) | 223, 296 |

| No. of Substance | Name of substance | Melting point (°C) | Specific rotatory power (°)* | | Elementary analytical Composition (%) C | H | N | Ultra violet absorption max.** |
|---|---|---|---|---|---|---|---|---|
| 11 | Benzyl m-aminobenzoate-N-L-fucoside | 162—164 | +51.6 | C=0.5 methanol | 64.3 (64.3 | 6.4 6.2 | 3.7 3.8) | 226, 246 and 325 |
| 12 | Benzyl m-aminobenzoate-N-lactoside | 204—207 (decomp.) | −50.0 | C=0.1 methanol | 56.3 (56.4 | 6.2 6.3 | 2.6 2.5) | 226, 246 and 325 |
| 13 | Benzyl p-aminobenzoate-N-D-deoxyriboside | — (syrup) | +34.6 | C=0.45 methanol | 66.4 (66.5 | 6.3 6.1 | 4.2 4.1) | 220, 290 |
| 14 | Benzyl p-aminobenzoate-N-D-glucoside | 88—95 | −43.2 | C=0.5 methanol | 61.8 (61.7 | 6.0 5.9 | 3.7 3.6) | 220, 290 |
| 15 | Benzyl p-aminobenzoate-N-D-fructoside | 90—98 | −148.0 | C=0.25 methanol | 62.1 (62.0 | 6.6 6.6 | 2.9 2.8) | 220, 290 |
| 16 | Cyclohexyl o-aminobenzoate-N-D-deoxyriboside | 153—155 | +3.2 | C=0.5 ethanol | 64.2 (64.3 | 7.5 7.7 | 4.2 4.2) | 222, 250 and 335 |
| 17 | Cyclohexyl m-aminobenzoate-N-D-mannoside | 161—166 | −40.0 | C=0.1 methanol | 59.8 (59.8 | 7.0 7.1 | 3.8 3.7) | 223, 245 |
| 18 | Cyclohexyl p-aminobenzoate-N-D-xyloside | 85—92 | −1.2 | C=0.5 methanol | 61.5 (61.5 | 7.2 7.1 | 4.1 4.0) | 220, 293 |
| 19 | Cyclohexyl p-aminobenzoate-N-cellobioside | 120—130 | −12.8 | C=0.5 methanol | 60.9 (60.9 | 7.1 7.1 | 2.7 2.8) | 220, 293 |
| 20 | Cyclohexyl p-aminobenzoate-N-maltotrioside | 158—165 | +68.4 | C=0.5 methanol | 52.8 (52.8 | 6.6 6.7 | 2.0 2.0) | 220, 293 |
| 21 | Hexahydrobenzyl m-aminobenzoate-N-lactoside | 147—157 | −2.8 | C=0.25 methanol | 56.0 (56.0 | 7.0 7.0 | 2.5 2.5) | 222, 250 and 337 |

TABLE 1 (Continued)

| No. of Substance | Name of substance | Melting point (°C) | Specific rotatory power (°)* | | Elementary analytical composition (%) C | H | N | Ultra violet absorption max.** |
|---|---|---|---|---|---|---|---|---|
| 22 | Hexahydrobenzyl m-aminobenzoate-N-L-fucoside | 115—123 | +43.6 | C=0.5 methanol | 63.1 (63.3 | 7.8 7.7 | 3.6 3.7) | 222, 245 |
| 23 | Hexahydrobenzyl p-aminobenzoate-N-D-riboside | 65—81 | +40.4 | C=0.5 methanol | 62.4 (62.5 | 7.4 7.4 | 3.8 3.8) | 221, 294 |
| 24 | Hexahydrobenzyl p-aminobenzoate-N-D-glucoside | 88—100 | −41.2 | C=0.5 methanol | 60.7 (60.8 | 7.3 7.3 | 3.5 3.5) | 221, 294 |
| 25 | Hexahydrobenzyl p-aminobenzoate-N-L-rhamnoside | 153—156 (decomp.) | +73.6 | C=0.5 ethanol | 63.3 (66.3 | 7.7 7.7 | 3.8 3.7) | 221, 294 |
| 26 | Methyl p-aminophenylacetate-N-L-rhamnoside | 88—100 | +48.4 | C=0.5 methanol | 57.7 (57.9 | 6.9 6.8 | 4.3 4.5) | 245 |
| 27 | p-Aminophenylacetic acid-N-L-rhamnoside | 137—139 | +16.7 | C=1 methanol | 56.31 (56.56 | 6.22 6.44 | 4.53 4.71) | 242, 288 |
| 28 | Sodium p-aminophenyl-acetate-N-L-rhamnoside | 144—146 | +15.9 | C=1 water | 52.75 (52.66 | 5.54 5.68 | 4.27 4.38) | 240, 287 |
| 29 | Potassium p-aminophenylacetate-N-L-rhamnoside | 134—137 | +17.4 | C=1 water | 50.10 (50.13 | 5.33 5.40 | 4.26 4.17) | 240, 288 |

Notes: * $[\alpha]_D^{20}$ (°), ** (nm), and *** theoretical composition

TABLE 2

Acute oral toxicities of the present substances ($LD_{50}$, $10^{-3}$kg/kg)

| No. of compound | $LD_{50}$ | No. of compound | $LD_{50}$ |
|---|---|---|---|
| 1 | 11.8 | 15 | 11.0 |
| 2 | larger than 15.0 | 16 | 8.8 |
| 3 | larger than 15.0 | 17 | larger than 15.0 |
| 4 | 13.6 | 18 | larger than 15.0 |
| 5 | 10.5 | 19 | 14.6 |
| 6 | larger than 15.0 | 20 | 12.5 |
| 7 | 14.8 | 21 | larger than 15.0 |
| 8 | 13.5 | 22 | 10.1 |
| 9 | larger than 15.0 | 23 | 12.6 |
| 10 | 12.4 | 24 | 12.1 |
| 11 | 9.7 | 25 | 9.4 |
| 12 | 11.5 | 26 | 11.2 |
| 13 | 9.2 | 27 | 13.8 |
| 14 | 11.7 | 28 | 11.1 |
| | | 29 | 10.0 |
| | | mitomycin C | 0.023 |

2) Anti-microbial activity:

The anti-microbial activity of the present substances was examined by culturing the following bacteria and fungi in respective culture media containing one of the twenty nine substances of Examples 1 to 29.

The microbes were:

Bacteria: *Pseudomonas aeruginosa,* strain IAM 1514
*Escherichia coli,* strain IFO 12734
*Staphylococcus aureus,* strain 209P
*Bacillus subtilis,* strain IAM 1069

Yeast: *Saccharomyces cerevisiae,* strain IAM 4207
*Candida albicans,* strain ATCC 752

Fungi: *Trichophyton mentagrophytes,* strain IFO 6124
*Aspergillus niger,* strain IAM 3001

Each culture medium was prepared as follows:

A series of 2 times-dilution of each substance were prepared by dissolution or suspension into distilled water, and they were respectively added to 9 times by weight of Heart-infusion agar medium (for bacteria) or of Sabouraud's agar medium (for fungi) each kept warm. These mixtures were poured into respective Petri dishes as culture plates. Preliminary cultured microbes were inoculated on the culture plates, and after culturing at 37°C for 20 to 24 hours (for bacteria) or at 25°C for 3 to 7 days (for fungi), the state of growth of the inoculated microbes was examined.

As the result, it was found that each substance of Examples 1 to 29 did not inhibit the microorganisms' growth at a concentration of 1 mg/ml.

3) Mutagenicity:

The mutagenicity of each of the 29 compounds of Examples 1 to 29 was examined by Rec-Assay,

namely growth inhibition to a recombination repair deficient strain M45 of *Bacillus subtilis,* and a recombination repair retaining strain H17 of *Bacillus subtilis,* cultured on B—II agar medium [consisting of $10^{-3}$ kg (1 g) of meat extract, $10^{-2}$ kg (10 g) of polypeptone, $5 \times 10^{-3}$ kg (5 g) of sodium chloride, $1.5 \times 10^{-2}$ kg (15 g) of agar and $10^{-3}m^3$ (1000 ml) of distilled water adjusted to pH of 7.0] by making the micro-organism's streaks not cross one another at their starting points on the culture plate. Then, a solution or suspension of each of the present compounds was absorbed on a round sheet of paper (disk) $8 \times 10^{-3}$m (8 mm) in diameter in an amount of $4 \times 10^{-8}m^3$ (0.04 ml). The disk was placed on the culture plate so as to cover the starting points of the streaks of the micro-organism. After culturing overnight at 37°C, the length of inhibited growth of the micro-organism was noted and compared with those caused by a negative control (kanamycin) and a positive control (mitomycin C). The results are shown in Table 3. As is seen in Table 3, no compound of the present invention showed a mutagenicity at a concentration as high as $5 \times 10^{-7}$ kg/disk (500 microgram/disk).

TABLE 3
Results of Rec-Assay of the present substances

| No. of Compound | Concentration ($10^{-9}$ kg/disk) | Length of growth inhibition | | Difference ($10^{-3}$m) |
|---|---|---|---|---|
| | | M45 ($10^{-3}$m) | (H17 ($10^{-3}$m) | |
| 1 | 500 | 0 | 0 | 0 |
| 2 | 500 | 0 | 0 | 0 |
| 3 | 500 | 0 | 0 | 0 |
| 4 | 500 | 0 | 0 | 0 |
| 5 | 500 | 0 | 0 | 0 |
| 6 | 500 | 0 | 0 | 0 |
| 7 | 500 | 0 | 0 | 0 |
| 8 | 500 | 0 | 0 | 0 |
| 9 | 500 | 0 | 0 | 0 |
| 10 | 500 | 0 | 0 | 0 |
| 11 | 500 | 0 | 0 | 0 |
| 12 | 500 | 0 | 0 | 0 |
| 13 | 500 | 0 | 0 | 0 |
| 14 | 500 | 0 | 0 | 0 |
| 15 | 500 | 0 | 0 | 0 |
| 16 | 500 | 0 | 0 | 0 |
| 17 | 500 | 0 | 0 | 0 |
| 18 | 500 | 0 | 0 | 0 |
| 19 | 500 | 0 | 0 | 0 |
| 20 | 500 | 0 | 0 | 0 |
| 21 | 500 | 0 | 0 | 0 |
| 22 | 500 | 0 | 0 | 0 |
| 23 | 500 | 0 | 0 | 0 |
| 24 | 500 | 0 | 0 | 0 |
| 25 | 500 | 0 | 0 | 0 |
| 26 | 500 | 0 | 0 | 0 |
| 27 | 500 | 0 | 0 | 0 |
| 28 | 500 | 0 | 0 | 0 |
| 29 | 500 | 0 | 0 | 0 |
| Kanamycin | 10 | 6 | 3 | 3 |
| Mitomycin C | 0.05 | 12 | 1 | 11 |

4) Delayed-type intracutaneous reaction:

In order to ascertain the influence of the present compounds on cellular immunity, a foot pad reaction test was carried out using ICR—JCL mice as experimental animals and sheep erythrocyte as an antigen as follows:

Sheep erythrocytes were suspended in a physiological saline solution at a concentration of 10% by volume. $2 \times 10^{-7}M^3$ (0.2 ml) of the suspension was injected into the caudal vein of each mouse for the primary sensitization. After 7 days, a 40% by volume suspension of the erythrocytes in a physiological saline solution was injected into the foot pad of each mouse for the challenge. The thickness of the injected foot pad was measured the next day. One of the present compounds was administered intraperitoneally at dose of $2.5 \times 10^{-4}$ kg/kg/day once a day for 5 days around the day of the first sensitization as a center.

The thickness of the foot pad of the mice to which any of the present compounds had been administered showed no significant difference as compared to that of a control animal (to which the present compound had not been administered). All the mice to which mitomycin C had been administered intraperitoneally at the same dose died and, accordingly, determination was not possible.

5) Production of antibody:

In order to ascertain the influence of the present compounds on the humoral immunity, the following test was carried out:

A rat was sensitized by injecting $2 \times 10^{-7}m^3$ (0.2 ml) of a suspension of sheep erythrocytes in physiological saline at a concentration of 10% by volume into the caudal vein. The blood of the mouse was collected after 7 days and tested by agglutination of the erythrocytes to ascertain the production of antibody. One of the present compounds was intraperitoneally administered for 5 days around the day of sensitization as a center. The results show that the agglutination values for the test animals were not significantly different from that of the control animal.

*Pharmaceutical properties:*

1) Anti-hyperglycemia activity (reduction of blood sugar level):

A group of Wistar rats, to which first streptozotocin had been interperitoneally administered at a rate of $6 \times 10^{-5}$ kg/kg (60 mg/kg) and which were glycosuria positive after a week of administration, and to which regular insulin was further administered and the reduction of the levels of glucose in urine and blood of which was once confirmed, but which again exhibited hyperglycemia and hyperglycosuria after a few days, was chosen and used as model animals for a glycosuria test as follows: After orally administering a respective one of the present compounds to the model animals at a dose of $3 \times 10^{-4}$ kg/kg (300 mg/kg) body weight, as a solution or dispersion in distilled water, blood was collected 3 and 6 hours after the administration. The level of glucose in the blood specimens was measured by an enzymatic method. The results are shown in Table 4.

TABLE 4
Activity of reducing the level of blood sugar

unit: $10^{-6}kg/10^{-4}m^3$ (mg/dl)

| No. of compound | Reduced level of blood sugar after | | No. of compound | Reduced level of blood sugar after | |
|---|---|---|---|---|---|
| | 3 hrs. | 6 hrs. | | 3 hrs. | 6 hrs. |
| 1 | 68 | 92 | 14 | 65 | 93 |
| 2 | 85 | 103 | 15 | 72 | 85 |
| 3 | 101 | 115 | 16 | 125 | 140 |
| 4 | 110 | 116 | 17 | 140 | 145 |
| 5 | 91 | 128 | 18 | 121 | 165 |
| 6 | 121 | 146 | 19 | 101 | 113 |
| 7 | 144 | 159 | 20 | 113 | 120 |
| 8 | 106 | 140 | 21 | 95 | 102 |
| 9 | 85 | 121 | 22 | 133 | 150 |
| 10 | 99 | 106 | 23 | 120 | 126 |
| 11 | 75 | 88 | 24 | 101 | 145 |
| 12 | 52 | 95 | 25 | 88 | 127 |
| 13 | 108 | 113 | 26 | 90 | 136 |
| | | | 27 | 91 | 90 |
| | | | 28 | 92 | 77 |
| | | | 29 | 88 | 71 |
| | | | Control | 21 | 19 |

2) Anti-hypertension activity (reduction of blood pressure):

A respective one of the present compounds was orally administered as a solution or dispersion in distilled water to rats with spontaneous hypertension at a dose rate of $3 \times 10^{-4}$ kg/kg (300 mg/kg) body weight. 3 and 6 Hours after the administration, the blood pressure of each rat was determined. The difference between these values and the value just before the administration was used as an index of the activity in reducing blood pressure of each compound. The results are shown in Table 5.

TABLE 5

Activity of reducing the blood pressure

unit: 133.3 Pa (mm Hg)

| No. of compound | Reducing level of blood pressure after | | No. of compound | Reduced level of blood pressure after | |
|---|---|---|---|---|---|
| | 3 hrs. | 6 hrs. | | 3 hrs. | 6 hrs. |
| 1 | 16 | 19 | 14 | 24 | 24 |
| 2 | 20 | 22 | 15 | 18 | 20 |
| 3 | 20 | 24 | 16 | 20 | 20 |
| 4 | 14 | 20 | 17 | 23 | 28 |
| 5 | 17 | 18 | 18 | 22 | 25 |
| 6 | 22 | 23 | 19 | 20 | 21 |
| 7 | 24 | 26 | 20 | 22 | 24 |
| 8 | 18 | 20 | 21 | 16 | 17 |
| 9 | 25 | 28 | 22 | 15 | 21 |
| 10 | 18 | 18 | 23 | 21 | 22 |
| 11 | 21 | 25 | 24 | 19 | 21 |
| 12 | 16 | 18 | 25 | 16 | 18 |
| 13 | 20 | 21 | 26 | 20 | 23 |
| | | | 27 | 20 | 20 |
| | | | 28 | 25 | 19 |
| | | | 29 | 21 | 17 |
| | | | Control | –2* | 0 |

3) Anti-tumour activity:

Sarcoma-180 was transplanted into the axillary part of ICR—JCL mice in an amount of $1 \times 10^6$ cells/animal. 24 Hours after transplantation, a respective one of the present compounds was orally administered as a solution or dispersion in a sterilized physiological saline solution to each of the mice 10 times every other day at a dose of $5 \times 10^{-4}$ kg/kg/time (500 mg/kg/time). On the 25th day after the transplantation, tumour node(s) were extirpated from all the mice to determine the weight of the nodes.

Antitumour activity (activity in inhibiting the proliferation of the transplanted sarcoma) of each of the present compounds was derived by the following formula and is shown in Table 6:

Antitumour activity (Inhibiting rate, I.R.) = $(1-T/C) \times 100$ (%).

wherein T represents the total weight of tumour nodes of the group of mice to which one of the present compounds had been administered, and

C represents the total weight of tumour nodes of the group of mice to which the present compounds were not administered.

TABLE 6
Antitumour activity against Sarcoma-180

| No. of compound | I.R. %* | No. of compound | I.R. % |
|---|---|---|---|
| 1 | 42 | 14 | 42 |
| 2 | 35 | 15 | 43 |
| 3 | 47 | 16 | 37 |
| 4 | 42 | 17 | 49 |
| 5 | 39 | 18 | 62 |
| 6 | 44 | 19 | 51 |
| 7 | 49 | 20 | 37 |
| 8 | 53 | 21 | 44 |
| 9 | 40 | 22 | 35 |
| 10 | 39 | 23 | 47 |
| 11 | 40 | 24 | 40 |
| 12 | 37 | 25 | 50 |
| 13 | 38 | 26 | 45 |
| | | 27 | 43 |
| | | 28 | 45 |
| | | 29 | 38 |

Note: *Inhibition rate or the rate of inhibiting the growth of the sarcoma.

4) Anti-hyperlipemia activity (reducing the level of lipids in blood):

Japanese white male rabbits were fed for 3 months with a solid diet containing 1% of cholesterol taken ad lib. After confirming a raised lipid content in the serum of the rabbits, they were utilized as model animals suffering from experimental arteriosclerosis. A respective one of the present compounds was administered once orally as a solution or dispersion in distilled water to groups of the experimental animals at a dose rate of $3 \times 10^{-4}$ kg/kg (300 mg/kg). Blood samples were taken at a predetermined time interval from the auricular vein of each animal. The content of cholesterol in the blood specimen was traced as the time passed by, with an enzymatic method, and the content of beta-lipoprotein in the same blood specimen was traced by colorimetry. The results are shown in Table 7.

Each value in Table 7 is the difference between the level just before administration and the level 3 or 6 hours after administration:

— means a rise in the cholesterol or beta-lipoprotein level.

TABLE 7

Anti-hyperlipemia activity (reducing the levels of total cholesterol and beta-lipoprotein in blood)

| No. of Compound | Reduction of cholesterol level $10^{-6}$ kg/$10^{-4}$m$^3$ (mg/dl) after | | Reduction of beta-lipoprotein level $10^{-6}$ kg/$10^{-4}$m$^3$ (mg/dl) after | |
|---|---|---|---|---|
| | 3 hrs. | 6 hrs. | 3 hrs. | 6 hrs. |
| 1 | –2 | 36 | 120 | 131 |
| 2 | 1 | 30 | 108 | 124 |
| 3 | 3 | 45 | 115 | 119 |
| 4 | 0 | 46 | 123 | 135 |
| 5 | –1 | 32 | 134 | 148 |
| 6 | 4 | 57 | 152 | 155 |
| 7 | –2 | 62 | 141 | 150 |
| 8 | 6 | 46 | 128 | 144 |
| 9 | –1 | 40 | 126 | 136 |
| 10 | –5 | 33 | 106 | 111 |
| 11 | –1 | 50 | 98 | 109 |
| 12 | 7 | 48 | 121 | 124 |
| 13 | 4 | 35 | 135 | 144 |
| 14 | 3 | 38 | 143 | 146 |
| 15 | –1 | 45 | 150 | 154 |
| 16 | 0 | 33 | 104 | 110 |
| 17 | –2 | 40 | 124 | 136 |
| 18 | 2 | 62 | 148 | 154 |
| 19 | 3 | 41 | 135 | 148 |
| 20 | –2 | 50 | 125 | 128 |
| 21 | 4 | 43 | 96 | 109 |
| 22 | 6 | 38 | 125 | 133 |
| 23 | –1 | 40 | 141 | 143 |
| 24 | 2 | 32 | 95 | 111 |
| 25 | –3 | 44 | 95 | 106 |
| 26 | –3 | 51 | 123 | 133 |
| 27 | 1 | 30 | 125 | 130 |
| 28 | 5 | 25 | 101 | 100 |
| 29 | 3 | 40 | 95 | 121 |
| Control | –3 | 0 | –1 | –4 |

5) Anti-inflammatory diseases:

5-1) Anti-carrageenin edema activity:

After orally administering a respective one of the present compounds once forcibly to groups of rats, composed of 10 animals, as a solution or dispersion in distilled water at a dose of $10^{-3}$ kg/kg (1000 mg/kg), $10^{-7}m^3$ (0.1 ml) of an aqueous dispersion of 1% carrageenin in an aqueous physiological saline solution was injected into the foot pad of the right hind leg of each animal following Van Arman *et al.* (1963). Thereafter, the thickness of the foot pad was measured after an interval of time to find the rate of inhibiting the swelling of the foot pad according to the following formula:

$$\text{Rate of inhibition (I.R.) (\%)} = (1 - T/C) \times 100$$

wherein T means the average volume of the foot pad of the animals to which one of the present compounds and carrageenin had been administered, and

C means the average volume of the foot pad of the animals to which carrageenin only and not one of the present compounds had been administered. The thus obtained I.R. (%) is shown in Table 8.

5-2) Anti-granuloma activity:

Following the method of Winter et al. (1963), two cotton wool pellets, each weighing $3 \times 10^{-5} \pm 10^{-6}$ kg (30 ± 1 mg) were implanted into the back of each rat of groups of rats, each group consisting of 6 animals, at the positions mutually symmetrical with respect to the median line of the rat. To these groups of rats, a respective one of the present compounds was administered orally and forcibly as a solution or dispersion in distilled water for 7 days, once a day, at a dose of $10^{-3}$ kg/kg/day (1000 mg/kg/day). On the 8th day after implantation, the granuloma was extirpated and the dry weight of the granuloma was measured. The rate of inhibiting the proliferation of granuloma was obtained as in 5-1) and is shown in Table 8.

5-3) Anti-exudation activity:

Following the method of Baris et al. (1965), air was injected subcutaneously into the backs of groups of rats, each group consisting of 6 animals, to make an air pouch. $5 \times 10^{-7}M^3$ (0.5 ml) of sesame oil containing 1% by weight of croton oil was injected into each air pouch. A respective one of the present compounds was orally administered forcibly to each of the groups of rats as a solution or dispersion in distilled water at a dose rate of $10^{-3}$ kg/kg/day (1000 mg/kg/day) for five days. The amount of liquid which exuded into the air pouch was measured on the 6th day after the operation. The rate of inhibiting the exudation of the liquid was obtained by the same manner as in 5-2) and the results are shown also in Table 8.

TABLE 8
Antiinflammatory activities of the present substances

Unit: Inhibiting Ratio (I.R.)%

| No. of compound | I.R. against carageenin edema | I.R. against cotton pellet edema | I.R. against exudation |
|---|---|---|---|
| 1 | 15.6 | 6.6 | 17.5 |
| 2 | 20.3 | 6.1 | 6.6 |
| 3 | 26.5 | 7.0 | 19.1 |
| 4 | 12.1 | 14.9 | 5.7 |
| 5 | 4.2 | 16.8 | 12.4 |
| 6 | 7.6 | 20.5 | 19.0 |
| 7 | 5.9 | 25.3 | 7.5 |
| 8 | 21.4 | 5.6 | 15.7 |
| 9 | 3.7 | 7.9 | 18.8 |
| 10 | 6.6 | 19.6 | 7.5 |
| 11 | 22.8 | 4.4 | 11.0 |
| 12 | 18.7 | 8.4 | 10.7 |
| 13 | 7.0 | 4.2 | 23.5 |
| 14 | 5.1 | 23.3 | 6.9 |
| 15 | 30.8 | 9.0 | 7.5 |
| 16 | 18.6 | 6.5 | 5.7 |
| 17 | 4.3 | 19.7 | 14.6 |
| 18 | 19.7 | 12.2 | 25.0 |
| 19 | 5.5 | 5.5 | 24.6 |
| 20 | 8.6 | 21.7 | 6.2 |
| 21 | 19.4 | 7.2 | 20.7 |
| 22 | 24.6 | 15.7 | 7.7 |
| 23 | 4.9 | 7.3 | 22.5 |
| 24 | 10.6 | 16.9 | 8.7 |
| 25 | 18.1 | 6.5 | 22.3 |
| 26 | 5.3 | 24.1 | 17.7 |
| 27 | 5.2 | 23.1 | 16.5 |
| 28 | 8.3 | 20.6 | 6.1 |
| 29 | 9.3 | 15.9 | 5.8 |
| Control | 0 | 0 | 0 |

6) Analgesic activity:

6-1) Against mechanical stimulation using pressure:

ICR female mice showing the threshold value of pain of 50 to 80 × 133.3 Pa (mmHg) when the base of their tails was subjected to pressure using the pressure-stimulation apparatus of Takagi and Kameyama *et al.* were chosen as the experimental animals. After administering orally $10^{-3}$ kg/kg (1000 mg/kg) of one of the present compounds, the abovementioned pressure test was repeatedly carried out as time passed to find the pressure and the time when the animal showed a pseudo-escaping reaction for evaluating the analgesic activity of the compound. The results are shown in Table 9.

6-2) Against chemical stimulation using acetic acid:

30 Minutes after oral administration of one of the present compounds at a dose of $10^{-3}$ kg/kg (1000 mg/kg) to a group of ICR female mice of 5 to 6 weeks after birth, a group being 10 animals, an aqueous 0.6% acetic acid solution was intraperitoneally injected into the mice in an amount of $10^{-7}m^3/10^{-2}$ kg (0.1 ml/10 g) of body weight of the mouse. The occurrence of writhing was counted for 10 minutes by the method of Kostet *et al.* The rate of inhibition of writhing was calculated by the following formula and is shown also in Table 9:

Rate of inhibition (I.R.) (%) = $(1-T/C) \times 100$, wherein T represents the average number of writhings of a group of mice to which one of the present compounds and acetic acid had been administered, and C represents the average number of writhings of a group of mice injected only with acetic acid (control).

The results are also shown in Table 9.

TABLE 9
Analgesic activity against physical stimulation

| No. of compound | Pseudo-escaping reaction | | I.R. of writhing (%) |
|---|---|---|---|
| | pressure × 133.3 Pa (mmHg) | time (sec) | |
| 1 | 78 | 38 | 17 |
| 2 | 84 | 42 | 20 |
| 3 | 93 | 44 | 22 |
| 4 | 96 | 45 | 41 |
| 5 | 90 | 41 | 52 |
| 6 | 98 | 43 | 37 |
| 7 | 95 | 44 | 46 |
| 8 | 80 | 39 | 20 |
| 9 | 86 | 38 | 28 |
| 10 | 86 | 36 | 30 |
| 11 | 74 | 35 | 17 |
| 12 | 90 | 42 | 33 |
| 13 | 82 | 40 | 24 |
| 14 | 92 | 44 | 39 |
| 15 | 78 | 37 | 19 |
| 16 | 87 | 39 | 26 |
| 17 | 92 | 42 | 47 |
| 18 | 94 | 39 | 33 |
| 19 | 82 | 37 | 24 |
| 20 | 75 | 35 | 17 |
| 21 | 84 | 40 | 28 |
| 22 | 75 | 37 | 19 |
| 23 | 80 | 39 | 23 |
| 24 | 85 | 42 | 41 |
| 25 | 93 | 44 | 50 |
| 26 | 92 | 41 | 34 |
| 27 | 91 | 40 | 35 |
| 28 | 87 | 38 | 25 |
| 29 | 84 | 41 | 26 |
| Control | 68 | 31 | 0 |

7) Anti-pyrexia activity:

Following the method of Winter *et al.* (1961), an aqueous dispersion of 20% by weight of *Saccharomyces cerevisiae* was subcutaneously injected into groups of rats, each group consisting of 6 animals. After 10 hours of fasting, $10^{-3}$ kg/kg (1000 mg/kg) of a respective one of the present compounds was orally administered as a solution or dispersion in distilled water to a rat. The rectall temperature of the rat was then measured after a predetermined interval of time to find the lowest temperature.

The anti-pyrexia activity of each compound was obtained from the following formula:

Anti-pyrexia activity represented by the rate of

$$\text{inhibition (I.R.) (\%)} = \frac{C_1 - T}{C_1 - C_2} \times 100,$$

wherein $C_2$ represents the average rectal temperature of a negative control which was treated with neither yeast nor one of the present compounds.

$C_1$ represents the average temperature of a positive control which was treated with yeast but not with one of the present compounds, and T represents the temperature of the tested animals, which were treated with both yeast and one of the present compounds.

The results are shown in Table 10. As is seen in Table 10, the present compounds exhibited the antipyretic activity.

TABLE 10

Anti-pyrexia activity of the present substances

| No. of compound | Rate of inhibiting pyrexia (I.R.) (%) | No. of compound | Rate of inhibiting pyrexia (I.R.) (%) |
|---|---|---|---|
| 1 | 41 | 14 | 56 |
| 2 | 36 | 15 | 26 |
| 3 | 20 | 16 | 48 |
| 4 | 28 | 17 | 30 |
| 5 | 59 | 18 | 62 |
| 6 | 69 | 19 | 37 |
| 7 | 31 | 20 | 23 |
| 8 | 47 | 21 | 28 |
| 9 | 32 | 22 | 29 |
| 10 | 41 | 23 | 15 |
| 11 | 29 | 24 | 48 |
| 12 | 17 | 25 | 54 |
| 13 | 39 | 26 | 60 |

As seen from the tabulated data of the pharmacological properties, each of the present compounds has a therapeutic activity against hyperglycemia, hypertension, arteriosclerosis, tumour, pain, pyrexia and inflammatory diseases. In addition, their toxicity to mammals is very low and therefore, any of the present compounds can be effectively applicable in treating the abovementioned diseases.

Where any of the present compounds is applied as a pharmaceutical for treating hyperglycemia, hypertension, tumours, arteriosclerosis, inflammatory diseases or stimulation of the central nerve system resulting in pain, it is possible to administer the compound in a state suitable for obtaining effectiveness corresponding to the kinds and symptoms of the diseases. The compound may be administered on its own or after formulation into a pharmaceutical composition with pharmaceutically

acceptable carriers, adjuvants or other pharmaceutical(s). The compound can be administered in unit dosage form.

As the unit dosage form, the present compounds can be in orally administrable forms such as powders, granules, tablets, sugar-coated tablets, capsules, syrups, spherical particles, suspensions, solutions or emulsions, and in parenterally administrable forms such as injections contained in ampoules or in vials and suppositories. The diluent may be solid, liquid or semisolid. Conventional carriers or diluents such as an excipient, vehicle, binding agent, wetting agent, disintegrating agent, surfactant, lubricant, dispersing agent, buffering agent, perfume, preservative, dissolution agent, solvent, etc. can be suitably utilized.

Concrete examples of the diluent (carrier and adjuvant) which may be exemplified are: lactose, sucrose, sorbitol, mannitol, starch, precipitated calcium carbonate, heavy magnesium oxide, talc, calcium stearate, magnesium stearate, cellulose and derivatives thereof, amylopectin, polyvinyl alcohol, gelatin, surfactants, water, aqueous physiological saline solution, ethanol, glycerin, propylene glycol, cacao butter, laurin fat, vaseline, paraffin, and higher alcohols.

The pharmaceutical composition containing the present compound as an active ingredient may be prepared by any known method. The content of the present compound in the pharmaceutical composition is generally 0.01 to 100% by weight, preferably from 0.05 to 80% by weight. The composition may be administered orally or parenterally. Preferably it is administered orally. Oral administration includes sublingual administration. Parenteral administration includes subcutaneous-, muscular-, intravenal- and drip administration, and rectal administration.

Since the dose rate of the present compound depends on species, age, sex, personal differences and the conditions of the disease, there may be cases where a larger or smaller amount of the present compound needs to be administered. However, generally in human cases, the oral dose rate is $10^{-7}$ to $5 \times 10^{-4}$ kg/kg (0.1 to 500 mg/kg) body weight/day, preferably $10^{-6}$ to $2.5 \times 10^{-4}$ kg/kg/day (1 to 250 mg/kg/day), and the parenteral dose rate is $10^{-8}$ to $2 \times 10^{-4}$ kg/kg/day (0.01 to 200 mg/kg/day), preferably, $10^{-7}$ to $10^{-4}$ kg/kg/day (0.1 to 100 mg/kg/day). These doses can be divided into 1 to 4 portions, one portion being administered at a time.

The present compounds and pharmaceutical compositions containing one of the present compounds are illustrated in the following Examples and Formulation Examples:

Example 1

Synthesis of o-aminobenzoic acid amide-N-D-fructoside:

$2.3 \times 10^{-3}$ Kg (2.3 g) of o-aminobenzoic acid amide, $2.7 \times 10^{-3}$ kg (2.7 g) of D-fructose and a few drops of concentrated hydrochloric acid were added to $3 \times 10^{-5}m^3$ (30 ml) of ethanol. The mixture was heated under a reflux condenser to bring the raw materials into condensation. Then the reaction mixture was condensed and poured into an excess acetone. The precipitate that separated was collected by filtration and dried to obtain a powdery substance, yellowish brown in colour, in a yield of 44%.

Example 2

Synthesis of m-aminobenzoic acid amide-N-cellobioside:

A solution containing $5.1 \times 10^{-3}$ kg (5.1 g) of cellobiose dissolved in $1.8 \times 10^{-5}m^3$ (18 ml) of water and a solution of $2.1 \times 10^{-3}$ kg (2.1 g) of m-aminobenzoic acid amide dissolved in $8 \times 10^{-6}m^3$ (8 ml) of ethanol were mixed. After adding $2 \times 10^{-6}m^3$ (2 ml) of acetic acid to the mixture, the whole mixture was heated under a reflux condenser to cause condensation. By repeated recrystallization of the reaction mixture from a mixture of water and ethanol, colourless and needle-like crystals were obtained in a yield of 26%.

Example 3

Synthesis of p-aminobenzoic acid amide-N-D-xyloside:

In $3 \times 10^{-5}m^3$ (30 ml) of an aqueous 94% ethanolic solution, $2.8 \times 10^{-3}$ kg (2.8 g) of p-aminobenzoic acid amide, $3.0 \times 10^{-3}$ kg (3.0 g) of D-xylose and $3 \times 10^{-4}$ kg (0.3 g) of ammonium chloride were heated under a reflux condenser to cause condensation. $10^{-5}M^3$ (10 ml) of the solvent was added during condensation to facilitate stirring. After cooling the reaction mixture, the separated crystals were repeatedly recrystallized from a mixture of water and methanol to obtain colourless needle-like crystals in a yield of 44%.

Example 4

Synthesis of p-aminobenzoic acid amide-N-D-mannoside:

In $2.5 \times 10^{-5}m^3$ (25 ml) of an aqueous 94% by weight of ethanolic solution, $2.1 \times 10^{-3}$ kg (2.1 g) of p-aminobenzoic acid amide, $2.5 \times 10^{-3}$ kg (2.5 g) of D-mannose and $2 \times 10^{-4}$ kg (0.2 g) of ammonium chloride were heated under a reflux condenser to obtain a white precipitate. The crystalline precipitate obtained by filtration of the reaction mixture was repeatedly recrystallized from an aqueous 94% ethanolic solution to obtain colourless needle-like crystals in a yield of 94%.

Example 5

Synthesis of p-aminobenzoic acid amide-N-L-rhamnoside:

In $2.5 \times 10^{-5} m^3$ (25 ml) of an aqueous 94% ethanolic solution, $2.3 \times 10^{-3}$ kg (2.3 g) of p-aminobenzoic acid amide, $2.7 \times 10^{-3}$ kg (2.7 g) of L-rhamnose and $2 \times 10^{-4}$ kg (0.2 g) of ammonium chloride were heated under a reflux condenser to cause condensation. The reaction mixture thus obtained was left overnight in an ice-box. The separated crystals were collected and subjected to repeated recrystallization from an aqueous 94% ethanolic solution to obtain colourless needle-like crystals in a yield of 68%.

Example 6

Synthesis of phenyl o-aminobenzoate-N-L-rhamnoside:

In $2.5 \times 10^{-5} m^3$ (25 ml) of an aqueous 94% ethanolic solution, $3.2 \times 10^{-3}$ kg (3.2 g) of phenyl o-aminobenzoate, $2.7 \times 10^{-3}$ kg (2.7 g) of L-rhamnose and $2 \times 10^{-4}$ kg (0.2 g) of ammonium chloride were heated under a reflux condenser to cause condensation. After condensing and cooling the reaction mixture, the separated crystals were collected and recrystallized repeatedly from an aqueous 94% ethanolic solution to obtain colourless needle-like crystals in a yield of 35.5%.

Example 7

Synthesis of phenyl m-aminobenzoate-N-D-mannoside:

In $2.5 \times 10^{-5} m^3$ (25 ml) of an aqueous 94% ethanolic solution, $3.0 \times 10^{-3}$ kg (3.0 g) of phenyl m-aminobenzoate, $2.5 \times 10^{-3}$ kg (2.5 g) of D-mannose and $2 \times 10^{-4}$ kg (0.2 g) of ammonium chloride were heated under a reflux condenser to cause condensation. The crystals which separated after cooling the reaction mixture were repeatedly recrystallized from an aqueous 94% ethanolic solution to obtain colourless needle-like crystals in a yield of 66.7%.

Example 8

Synthesis of phenyl p-aminobenzoate-N-D-xyloside:

In $3 \times 10^{-5} m^3$ (30 ml) of an aqueous 94% ethanolic solution, $3.2 \times 10^{-3}$ kg (3.2 g) of phenyl p-aminobenzoate, $2.3 \times 10^{-3}$ kg (2.3 g) of D-xylose and $2 \times 10^{-4}$ kg (0.2 g) of ammonium chloride were heated under a reflux condenser. After condensing the reaction mixture to $\frac{1}{3}$ its original volume, the condensate was shaken with an addition of ether and water. The aqueous layer that separated was extracted with ethyl acetate repeatedly and the layer of ethyl acetate was condensed under reduced pressure and dried to obtain a yellowish brown powdery material as the object in a yield of 23%.

Example 9

Synthesis of phenyl p-aminobenzoate-N-cellobioside:

An aqueous solution of $5.1 \times 10^{-3}$ kg (5.1 g) of cellobiose dissolved in $1.3 \times 10^{-5} m^3$ (13 ml) of water and a solution of $3.2 \times 10^{-3}$ kg (3.2 g) of phenyl p-aminobenzoate dissolved in $10^{-5} m^3$ (10 ml) of ethanol were mixed. After adding $3.3 \times 10^{-6} m^3$ (3.3 ml) of acetic acid to the mixture, the whole mixture was heated under a reflux condenser to bring the raw materials into condensation. After condensing and leaving the reaction mixture alone, the separated crystals of cellobiose were removed. The filtrate was subjected to thin layer chromatography and eluted by a solvent mixture of methanol, benzene, butanol and water at a ratio of 5:5:10:2 by volume. The eluate was again subjected to chromatography to purify the product and a slightly yellowish powdery substance was obtained in a yield of 12.5%.

Example 10

Synthesis of phenyl p-aminobenzoate-N-maltotrioside:

An aqueous solution of $5.0 \times 10^{-3}$ kg (5.0 g) of maltotriose dissolved in $2.8 \times 10^{-6} m^3$ (2.8 ml) of water and a solution of $2.1 \times 10^{-3}$ kg (2.1 g) of phenyl p-aminobenzoate dissolved in $8.5 \times 10^{-6} m^3$ (8.5 ml) of ethanol were mixed. After adding $1.4 \times 10^{-6} m^3$ (1.4 ml) of acetic acid to the mixture, the whole mixture was heated under a reflux condenser to bring the raw materials into condensation. The reaction mixture was condensed to dryness and dissolved in an aqueous 50% ethanolic acid. This solution was poured into excess acetone to separate the desired product out from the mixture. The precipitate was dissolved into an aqueous 50% ethanolic solution. This solution was subjected to thin layer chromatography using a silicagel plate. The adsorbed product was eluted by a solvent mixture of methanol, benzene, butanol and water at a volume ratio of 5:5:10:2. The eluate was again subjected to the same chromatography to separate and obtain the product as a pale yellow powdery substance in a yield of 5.2%.

Example 11

Synthesis of benzyl m-aminobenzoate-N-L-fucoside:

In $2 \times 10^{-5} m^3$ (20 ml) of an aqueous 94% ethanolic solution, $2.1 \times 10^{-3}$ kg (2.1 g) of benzyl m-aminobenzoate, $1.5 \times 10^{-3}$ kg (1.5 g) of L-fucose and $2 \times 10^{-4}$ kg (0.2 g) of ammonium chloride were heated under a reflux condenser to bring the raw materials into condensation, and the reaction mixture was condensed. The condensate was poured into excess acetone, and the insoluble matter was

removed by filtration. The filtrate was evaporated to dryness. After dissolving the dried solid into an aqueous 50% ethanolic solution, the solution was subjected to thin layer chromatography using a silicagel plate. The adsorbed substance was eluted by a solvent mixture of methanol, benzene, butanol and water at a volume ratio of 5:5:10:2. The eluate was again subjected to the same chromatography to obtain the desired product as a pale yellow powdery material in a yield of 19%.

Example 12

Synthesis of benzyl m-aminobenzoate-N-lactoside:

An aqueous solution of 3.6 x $10^{-3}$ kg (3.6 g) of lactose dissolved in $10^{-5}m^3$ (10 ml) of water and a solution of 2.3 x $10^{-3}$ kg (2.3 g) of benzyl m-aminobenzoate dissolved in 5.5 x $10^{-6}m^3$ (5.5 ml) of ethanol were mixed. After adding 1.4 x $10^{-6}m^3$ (1.4 ml) of acetic acid to the mixture, the whole mixture was heated under a reflux condenser to bring the raw materials into condensation. After cooling the reaction mixture, the thus separated precipitate was collected by filtration and recrystallized several times from a solvent mixture of methanol, water and dioxane to obtain needle-like crystals in a yield of 22.7%.

Example 13

Synthesis of benzyl p-aminobenzoate-N-D-deoxyriboside:

In 2.5 x $10^{-5}m^3$ (25 ml) of an aqueous 94% ethanolic solution, 3.4 x $10^{-3}$ kg (3.4 g) of benzyl p-aminobenzoate, 2.0 x $10^{-3}$ kg (2.0 g) of D-deoxyribose and 2 x $10^{-4}$ kg (0.2 g) of ammonium chloride were heated under a reflux condenser to bring the raw materials into condensation, and the reaction mixture was condensed and poured into acetone. After filtrating the mixture, the filtrate was dried to solid. Then, the solid was subjected to procedures similar to those of Example 11 to obtain a yellowish brown syrup in a yield of 10%.

Example 14

Synthesis of benzyl p-aminobenzoate-N-D-glucoside:

In a similar manner to Example 13, except using 2.7 x $10^{-3}$ kg (2.7 g) of D-glucose in 3 x $10^{-5}m^3$ (30 ml) of an aqueous 94% ethanolic solution instead of 2.0 x $10^{-3}$ kg (2.0 g) of D-deoxyribose in 2.5 x $10^{-5}m^3$ (25 ml) of the solvent, a faintly yellow powdery substance was obtained in a yield of 9.4%.

Example 15

Synthesis of benzyl p-aminobenzoate-N-D-fructoside:

In a similar manner to Example 14, except for using D-fructose instead of D-glucose, a condensation reaction was carried out. The reaction mixture was condensed to dryness. The dried solid was dissolved in methanol while heating. After separating the crystals which precipitated on cooling the solution, the filtrate was condensed, and extracted with water and ethyl acetate. After condensing the organic layer, the condensate was subjected to thin layer chromatography using a silicagel plate while eluting with a solvent mixture of methanol, benzene, butanol and water at a volume ratio of 5:5:10:2. The eluate was again subjected to the same chromatography to obtain a yellow powdery substance in a yield of 2%.

Example 16

Synthesis of cyclohexyl o-aminobenzoate-N-D-deoxyriboside:

In 2.5 x $10^{-3}m^3$ (25 ml) of an aqueous 94% ethanolic solution, 3.3 x $10^{-3}$ kg (3.3 g) of cyclohexyl o-aminobenzoate, 2.0 x $10^{-3}$ kg (2.0 g) of D-deoxyribose and 2 x $10^{-4}$ kg (0.2 g) of ammonium chloride were heated under a reflux condenser to bring the raw material into condensation. The reaction mixture was dried to a solid. The solid was dissolved in methanol by heating. After collecting the crystals precipitated on cooling the methanolic solution, the crystals were repeatedly recrystallized from a mixture of methanol, acetone and water to obtain colourless and needle-like crystals in a yield of 17.9%.

Example 17

Synthesis of cyclohexyl m-aminobenzoate-N-D-mannoside:

In 2.5 x $10^{-5}m^3$ (25 ml) of an aqueous 94% ethanolic solution, 3.1 x $10^{-3}$ kg (3.1 g) of cyclohexyl m-aminobenzoate, 2.5 x $10^{-3}$ kg (2.5 g) of D-mannose and 2 x $10^{-4}$ kg (0.2 g) of ammonium chloride were heated under a reflux condenser to bring the raw materials into condensation. After letting the reaction mixture cool, the precipitated crystals were collected by filtration and repeatedly recrystallized from an aqueous 94% ethanolic solution to obtain flaky crystals in a yield of 91.6%.

Example 18

Synthesis of cyclohexyl p-aminobenzoate-N-D-xyloside:

In 3 x $10^{-5}m^3$ (30 ml) of an aqueous 94% ethanolic solution, 3.3 x $10^{-3}$ kg (3.3 g) of cyclohexyl p-aminobenzoate, 2.3 x $10^{-3}$ kg (2.3 g) of D-xylose and 2 x $10^{-4}$ kg (0.2 g) of ammonium chloride

were heated under a reflux condenser to bring the raw materials into condensation. After condensing the reaction mixture, the condensate was poured into an excess acetone followed by removing the thus precipitated matter by filtration. The filtrate was dried to solid and the solid was dissolved in an aqueous 50% methanolic solution, the solution being subjected to thin layer chromatography as in Example 9 to obtain a pale yellow powdery substance in a yield of 8%.

Example 19

Synthesis of cyclohexyl p-aminobenzoate-N-cellobioside:

An aqueous solution of $5.1 \times 10^{-3}$ kg (5.1 g) of cellobiose dissolved in $1.3 \times 10^{-5}m^3$ (13 ml) of water and a solution of $3.3 \times 10^{-3}$ kg (3.3 g) of cyclohexyl p-aminobenzoate dissolved in $10^{-5}m^3$ (10 ml) of ethanol were mixed. After adding $3.3 \times 10^{-6}m^3$ (3.3 ml) of acetic acid to the mixture, the whole mixture was heated under a reflux condenser to bring the raw materials into condensation. The reaction mixture was treated as in Example 11 to obtain a pale yellow powdery substance in a yield of 4.9%.

Example 20

Synthesis of cyclohexyl p-aminobenzoate-N-maltotrioside:

An aqueous solution of $5.0 \times 10^{-3}$ kg (5.0 g) of maltotriose dissolved in $4 \times 10^{-6}m^3$ (4 ml) of water and a solution of $2.2 \times 10^{-3}$ kg (2.2 g) of cyclohexyl p-aminobenzoate dissolved in $8.5 \times 10^{-6}m^3$ (8.5 ml) of ethanol were mixed. After adding $1.4 \times 10^{-6}m^3$ (1.4 ml) of acetic acid to the mixture, the whole mixture was heated under a reflux condenser to bring the raw materials into condensation. After drying the reaction mixture to solid, and dissolving the solid into a solvent mixture of methanol and water, the solution was poured into an excess acetone. After collecting the thus separated precipitate by filtration, the precipitate was dissolved into methanol. This solution was subjected to thin layer chromatography as in Example 9 to obtain a yellow powdery substance in a yield of 11%.

Example 21

Synthesis of hexahydrobenzyl o-aminobenzoate-N-lactoside:

An aqueous solution of $3.6 \times 10^{-3}$ kg (3.6 g) of lactose dissolved in $1.2 \times 10^{-5}m^3$ (12 ml) of water and a solution of $2.3 \times 10^{-3}$ kg (2.3 g) of hexahydrobenzyl o-aminobenzoate dissolved in $4 \times 10^{-6}m^3$ (4 ml) of methanol were mixed. After adding $1.4 \times 10^{-6}m^3$ (1.4 ml) of acetic acid to the mixture, the whole mixture was heated under a reflux condenser to bring the raw materials into condensation. During the reaction, since the reaction mixture separated into 2 phases, a small amount of dioxane was added to prevent this separation. After condensing the reaction mixture to dryness, the dried material was extracted with water and a large amount of chloroform. After drying the chloroform layer to solid and extracting the solid with water and ethyl ether, the aqueous layer was extracted with ethyl acetate. The extract was condensed to dryness to obtain a yellowish brown powdery substance in a yield of 1.4%.

Example 22

Synthesis of hexahydrobenzyl m-aminobenzoate-N-L-fucoside:

In $2 \times 10^{-5}m^3$ (20 ml) of an aqueous 94% ethanolic solution, $2.1 \times 10^{-3}$ kg (2.1 g) of hexahydrobenzyl m-aminobenzoate, $1.5 \times 10^{-3}$ kg (1.5 g) of L-fucose and $2 \times 10^{-4}$ kg (0.2 g) of ammonium chloride were heated under a reflux condenser to bring the raw materials into condensation. The reaction mixture was then condensed to dryness. The dried matter was subjected to the same procedures, including thin layer chromatography, as in Example 10, to obtain a pale yellow powdery substance in a yield of 24.5%.

Example 23

Synthesis of hexahydrobenzyl p-aminobenzoate-N-D-riboside:

In $2.5 \times 10^{-5}m^3$ (25 ml) of an aqueous 94% ethanolic solution, $3.5 \times 10^{-3}$ kg (3.5 g) of hexahydrobenzyl p-aminobenzoate, $2.3 \times 10^{-3}$ kg (2.3 g) of D-ribose and $2 \times 10^{-4}$ kg (0.2 g) of ammonium chloride were heated under a reflux condenser to bring the raw materials into condensation. After condensing the reaction mixture to dryness, the dried material was subjected to the same procedures as in Example 22 to obtain a pale yellow powdery substance in a yield of 5.4%.

Example 24

Synthesis of hexahydrobenzyl p-aminobenzoate-N-D-glucoside:

The same condensation was carried out as in Example 23 except for using $2.7 \times 10^{-3}$ kg (2.7 g) of D-glucose in $3 \times 10^{-5}m^3$ (30 ml) of 94% ethanolic solution instead of $2.3 \times 10^{-3}$ kg (2.3 g) of D-ribose in $2.5 \times 10^{-5}m^3$ (25 ml) of 94% ethanolic solution. The reaction mixture was evaporated to dryness. The dried material was dissolved in an aqueous methanolic solution. After pouring the methanolic solution into excess acetone and removing the thus separated precipitate by filtration, the filtrate was evaporated to dryness. The dried material was dissolved in methanol. The solution was subjected to thin layer chromatography as in Example 9 to obtain a pale yellow substance in a yield of 11.5%.

Example 25

Synthesis of hexahydrobenzyl p-aminobenzoate-N-L-rhamnoside:

The same condensation was carried out as in Example 24, except that for using $2.7 \times 10^{-3}$ kg (2.7 g) of L-rhamnose in $2 \times 10^{-5}m^3$ (25 ml) of the 94% ethanolic solution instead of $2.7 \times 10^{-3}$ kg (2.7g) of D-glucose in $3 \times 10^{-5}m^3$ (30 ml) of the ethanolic solution. After condensing the reaction mixture, and cooling the condensate, the thus separated crystals were recrystallized from the 94% ethanolic solution repeatedly to obtain colourless and needle-like crystals in a yield of 82%.

Example 26

Synthesis of methyl p-aminophenylacetate-N-L-rhamnoside:

The same condensation was carried out as in Example 25, except for using $2.5 \times 10^{-3}$ kg (2.5 g) of methyl p-aminophenylacetate instead of $3.5 \times 10^{-3}$ kg (3.5 g) of hexahydro p-aminobenzoate. After condensing the reaction mixture and pouring the condensate into excess acetone, the thus separated precipitate was removed by filtration. The filtrate was evaporated to form a syrup. After washing the syrup repeatedly with benzene, the washed material was dissolved in $2 \times 10^{-5}m^3$ (20 ml) of ethanol. By adding $5 \times 10^{-6}m^3$ (5 ml) of hexane to the ethanolic solution, a crystalline substance, the desired product, was isolated and dried to a yield of 45%.

Example 27

Synthesis of p-aminophenylacetic acid-N-L-rhamnoside:

Into ethanol, $1.51 \times 10^{-3}$ kg (1.51 g) of p-aminophenylacetic acid and $1.82 \times 10^{-3}$ kg (1.82 g) of L-rhamnose monohydrate were dissolved. After adding $5.4 \times 10^{-5}$ kg (54 mg) of ammonium chloride to the solution, the mixture was heated for 10 minutes under a reflux condenser in a stream of gaseous nitrogen. After filtering the reaction mixture using a sheet of filter paper, the filtrate was cooled. Then, the deposited crystals were collected by filtration and dried. The yield was $1.67 \times 10^{-3}$ kg (1.67 g). Purified p-aminophenylacetic acid-N-L-rhamnoside was obtained by recrystallizing the dried crystals with ethanol.

Example 28

Synthesis of sodium p-aminophenylacetate-N-L-rhamnoside:

Into $3 \times 10^{-6}m^3$ (3 ml) of water containing $4 \times 10^{-5}$ kg (40 mg) of sodium hydroxide, $2.973 \times 10^{-4}$ kg (297.30 mg) of p-aminophenylacetic acid-N-L-rhamnoside obtained in Example 27 was dissolved. After condensing the solution and dissolving the condensate in $10^{-6}m^3$ (1 ml) of methanol, the mixture was dispersed into a large amount of acetone. Then, the deposited crystals were collected by filtration and dried to obtain $1.5 \times 10^{-4}$ kg (150 mg) of sodium p-aminophenylacetate-N-L-rhamnoside. Purification of the product was carried out by dissolving the crystals in methanol and dispersing the solution in a large amount of acetone.

Example 29

Synthesis of potassium p-aminophenylacetate-N-L-rhamnoside:

Into $3 \times 10^{-6}m^3$ (3 ml) of water containing $5.6 \times 10^{-5}$ kg (56 mg) of potassium hydroxide, $2.973 \times 10^{-4}$ kg (297.30 mg) of p-aminophenylacetic acid-N-L-rhamnoside obtained in Example 27 was dissolved. After condensing the solution and dissolving the condensate into $10^{-6}m^3$ (1 ml) of methanol, the mixture was dispersed into a large amount of acetone. Then, the deposited crystals were collected by filtration and dried to obtain $1.3 \times 10^{-4}$ kg (130 mg) of potassium p-aminophenylacetate-N-L-rhamnoside. Purification of the crystals was carried out in the same manner as in Example 28.

Formulation Example 1

One of the present substances, i.e. benzyl p-aminobenzoate-N-D-deoxyriboside (substance No. 13), heavy magnesium oxide and lactose were well mixed to form a uniform powdery material or uniform minute particles at a weight ratio of 10:15:75. The composition was sifted to collect the fraction smaller than $3.5 \times 10^{-4}$m (350 $\mu$) in size to form a powdery composition. Capsules were separately prepared by encapsulating this powdery composition.

Formulation Example 2

One of the present substances, i.e. p-aminobenzoic acid amide-N-D-mannoside (substance No. 4), starch, lactose, crystalline cellulose, polyvinyl alcohol and water were uniformly mixed at a weight ratio of 45:15:16:21:3:30. After crushing, granulating and drying the mixture, the product was sifted to collect the fraction of $1.77 \times 10^{-4}$ to $1.41 \times 10^{-3}$m (177 to 1410 $\mu$) in size. The thus obtained product was a granular composition.

Formulation Example 3

A granular composition was prepared in the same manner as in Formulation Example 2, except for using phenyl o-aminobenzoate-N-L-rhamnoside (substance No. 6) instead of p-aminobenzoic acid amide-N-D-mannoside. Tablets were prepared separately by adding calcium stearate at a ratio of 4:96 to the granules and compressing the mixture into tablets $10^{-2}$m (10 mm) in diameter.

Formulation Example 4

Ten parts by weight of crystalline cellulose and 3 parts by weight of calcium stearate were added to 90 parts by weight of the granules prepared in Formulation Example 2. After compressing the mixture to form tablets 8 x $10^{-3}$m (8 mm) in diameter, a mixed suspension of syrup, gelatin and precipitated calcium carbonate was added to the tablets to prepare sugar-coated tablets.

Formulation Example 5

A mixture of 0.6 part by weight of m-aminobenzoic acid amide-N-cellobioside (Substance No. 2), 2.4 parts by weight of a non-ionic surfactant and 97 parts by weight of an aqueous physiological saline solution was prepared while warming the components. The mixture was dividedly introduced into ampoules and sterilized for injection.

**Claims**

1. A saccharide derivative represented by the general formula (I)

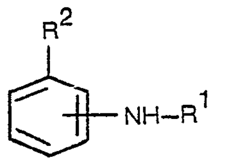

(I)

wherein $R^1$ represents a residual group formed by removing the hemiacetal hydroxyl group from a monosaccharide, disaccharide or trisaccharide, an aminosaccharide, D-glucuronic acid or L-gulonic acid; $R^2$ represents

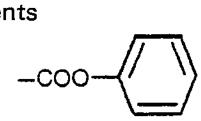 , 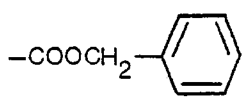 , 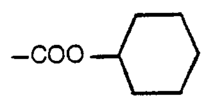 ,

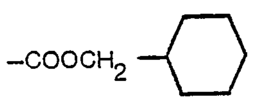 , $-CONH_2$ or $-CH_2COOR^3$

and $R^3$ represents a hydrogen atom, an alkyl of one to four carbon atoms or one equivalent of pharmaceutically acceptable metal.

2. A saccharide derivative according to claim 1, wherein $R^1$ is the group formed by removing the hemiacetal hydroxyl group from ribose, deoxyribose, glucose, fructose, xylose, mannose, rhamnose, fucose, lactose, cellobiose or maltotriose.

3. A saccharide derivative according to claim 1, wherein, in the case where $R^2$ of formula (I) represents —$CH_2COOR^3$, $R^3$ is Na, K, 1/2Ca, 1/2Mg, 1/3Al, methyl-, ethyl-, propyl- or butyl group.

4. A saccharide derivative according to claim 1 which is o-aminobenzoic acid amide-N-D-fructoside, m-aminobenzoic acid amide-N-cellobioside, p-aminobenzoic acid amide-N-D-xyloside, p-aminobenzoic acid amide-N-D-mannoside, or p-aminobenzoic acid amide-N-L-rhamnoside.

5. A saccharide derivative according to claim 1 which is phenyl o-aminobenzoate-N-L-rhamnoside, phenyl m-aminobenzoate-N-D-mannoside, phenyl p-aminobenzoate-N-D-xyloside, phenyl p-aminobenzoate-N-cellobioside, or phenyl p-aminobenzoate-N-maltotrioside.

6. A saccharide according to claim 1 which is benzyl m-aminobenzoate-N-L-fucoside, benzyl m-aminobenzoate-N-lactoside, benzyl p-aminobenzoate-N-D-deoxyriboside, benzyl p-aminobenzoate-N-D-glucoside or benzyl p-aminobenzoate-N-D-fructoside.

7. A saccharide according to claim 1 which is cyclohexyl o-aminobenzoate-N-D-deoxyriboside, cyclohexyl m-aminobenzoate-N-D-mannoside, cyclohexyl p-aminobenzoate-N-D-xyloside, cyclohexyl p-aminobenzoate-N-cellobioside, cyclohexyl p-aminobenzoate-N-maltotrioside, hexahydrobenzyl m-aminobenzoate-N-lactoside, hexahydrobenzyl m-aminobenzoate-N-L-fucoside, hexahydrobenzyl p-aminobenzoate-N-D-riboside, hexahydrobenzyl p-aminobenzoate-N-D-glucoside, or hexahydrobenzyl p-aminobenzoate-N-L-rhamnoside.

8. A saccharide according to claim 1 which is methyl p-aminophenylacetate-N-L-rhamnoside, p-aminophenylacetic acid-N-L-rhamnoside, sodium p-aminophenylacetate-N-L-rhamnoside, or potassium p-aminophenylacetate-N-L-rhamnoside.

9. A process for the preparation of a saccharide derivative as claimed in claim 1, which process comprises reacting a compound of formula [II]

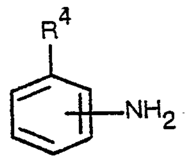

[II]

wherein $R^4$ represents 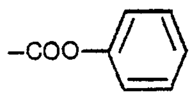, 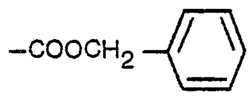, 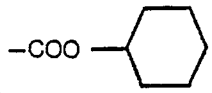,

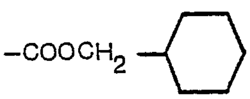, $-CONH_2$ or $-CH_2COOR^5$

and $R^5$ represents a hydrogen atom or a lower alkyl group, with a mono-, di- or trisaccharide, an aminosaccharide, D-glucuronic or L-gulonic acid and, if desired, converting a resulting compound wherein $R^4$ represents $—CH_2COOH$ to a compound wherein $R^4$ represents $—CH_2COOR^6$, $R^6$ representing an equivalent of a pharmaceutically acceptable metal.

10. A pharmaceutical composition comprising a saccharide derivative as claimed in any one of claims 1 to 8 or which has been produced by a process as claimed in claim 9 as active ingredient, together with a pharmaceutically acceptable carrier or diluent.

**Revendications**

1. Un dérivé de saccharide représenté par la formule générale (I)

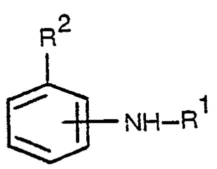

(I)

dans laquelle $R^1$ représente un groupe résiduel formé en éloignant le groupe hydroxyle hémiacétal d'un monosaccharide, disaccharide ou trisaccharide, un aminosaccharide, acide D-glucoronique ou acide L-gulonique; $R^2$ représente

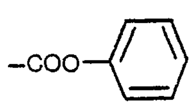, 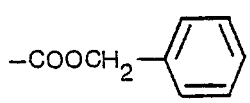, 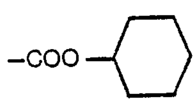,

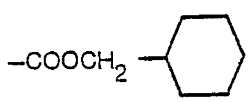, $-CONH_2$ or $-CH_2COOR^3$

$R^3$ représentant un atome hydrogène, un alkyle d'un à quatre atomes carbone ou un équivalent de métal pharmaceutiquement acceptable.

2. Un dérivé de saccharide selon la revendication 1, caractérisé en ce que $R^1$ est le groupe formé en éloignant le groupe hydroxyle hémiacétal de ribose, déoxyribose, glucose, fructose, xylose, mannose, rhamnose, fucose, lactose, cellobiose, ou maltoriose.

3. Un dérivé de saccharide selon la revendication 1, caractérisé en ce que, dans le cas où $R^2$ de la formule (I) représente $—CH_2COOR^3$, $R^3$ est Na, K, 1/2Ca, 1/2Mg, 1/3Al, un groupe méthylique, éthylique, propylique ou butylique.

4. Un dérivé de saccharide selon la revendication 1, caractérisé en ce qu'il est de l'acide aminobenzoïque-o fructoside-N-D amide, de l'acide aminobenzoïque-m cellobioside-N-amide, de l'acide aminobenzoïque-p xyloside-N-D-amide, de l'acide aminobenzoïque-p mannoside-N-D-amide, ou de l'acide aminobenzoïque-p rhamnoside-N-L-amide.

5. Un dérivé de saccharide selon la revendication 1, caractérisé en ce qu'il est du phényl rhamnoside-N-L-aminobenzoate-o, du phényl mannoside-N-D-aminobenzoate-m, du phényl xyloside-N-D-aminobenzoate, du phényl cellobioside-N- aminobenzoate-p, ou du phényl maltotrioside-N-aminobenzoate-p.

6. Un dérivé de saccharide selon la revendication 1, caractérisé en ce qu'il est du benzyl fucoside-N-L- aminobenzoate-m, du benzyl lactoside-N- aminobenzoate-m, du benzyl déoxyriboside-N-D-aminobenzoate-p, du benzyl glucoside-N-D- aminobenzoate-p ou du benzyl fructoside-N-D- aminobenzoate-p.

7. Un dérivé de saccharide selon la revendication 1, caractérisé en ce qu'il est du cyclohexyl déoxyriboside-N-D-aminobenzoate-o, du cyclohexyl mannoside-N-D-aminobenzoate-m, du cyclohexyl xyloside-N-D-aminobenzoate-p, du cyclohexyl cellobioside-N-aminobenzoate-p, du cyclohexyl maltorioside-N-aminobenzoate-p, de l'hexahydrobenzyl lactoside-N-aminobenzoate-m, de l'hexahydrobenzyl fucoside-N-L-aminobenzoate-m, de l'hexahydrobenzyl riboside-N-D-aminobenzoate-p, de l'hexahydrobenzyl glucoside-N-D-aminobenzoate-p, ou de l'hexahydrobenzyl rhamnoside-N-L-aminobenzoate-p.

8. Un dérivé de saccharide selon la revendication 1, caractérisé en ce qu'il est du méthyl rhamnoside-N-L-aminophénylacétate-p, de l'acide N-L-rhamnoside amino-phénylacétique-p, du sodium rhamnoside-N-L-aminophénylacétate-p, ou du potassium rhamnoside-N-L-aminophénylacétate-p.

9. Un procédé pour la préparation d'un dérivé de saccharide comme revendiqué dans la revendication 1, caractérisé en ce qu'il comprend la mise en réaction d'un composé de formule (II)

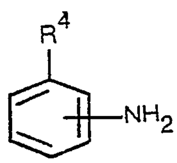

(II)

formule dans laquelle $R^4$ représente 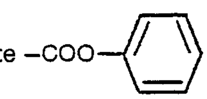 , 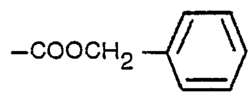 , 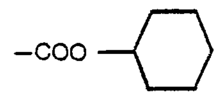 ,

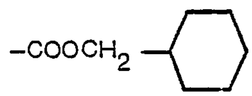 , $-CONH_2$ or $-CH_2COOR^5$

et $R^5$, représente un atome hydrogène ou un groupe alkyl bas, avec un mono-, di- ou trisaccharide, un a aminosaccharide, acide D-glucoronique ou acide L-gulonique, et si on le désire, en convertissant un composé ainsi obtenu dans lequel $R^4$ représente —$CH_2COOH$ en un composé dans lequel $R^4$ représente —$CH_2COOR^6$, $R^6$ représentant un équivalent d'un métal pharmaceutiquement acceptables.

10. Une composition pharmaceutique comprenant un dérivé de saccharide comme revendiqué dans une quelconque des revendications 1 à 8 ou qui a été produite comme ingrédient actif par un procédé tel que celui revendiqué dans la revendication 9, conjointement avec un excipient ou diluant pharmaceutiquement acceptable.

**Patentansprüche**

1. Saccharidderivat der allgemeinen Formel (I)

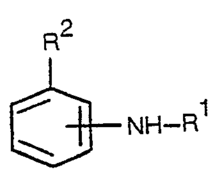

worin $R^1$ für eine Restgruppe steht, gebildet durch Entfernung der hemiacetalen Hydroxylgruppe aus einem Monosaccharid, Disaccharid oder Trisaccharid, einem Aminosaccharid, einer D-Glucuronsäure oder L-Gulonsäure, $R^2$

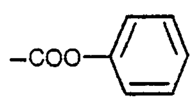 , 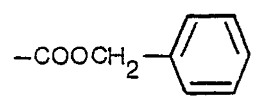 , 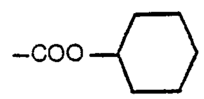 ,

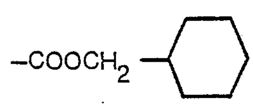 , $-CONH_2$ or $-CH_2COOR^3$

bedeutet und $R^3$ ein Wasserstoffatom, eine Alkylgruppe mit 1 bis 4 Kohlenstoffatomen oder 1 Äquivalent eines pharmazeutisch verträglichen Metalls darstellt.

2. Saccharidderivat nach Anspruch 1, dadurch gekennzeichnet, daß $R^1$ eine Gruppe ist, gebildet durch Entfernung der hemiacetalen Hydroxylgruppe aus Ribose, Deoxyribose, Glucose, Fructose, Xylose, Mannose, Rhamnose, Fucose, Lactose, Cellobiose oder Maltotriose.

3. Saccharidderivat nach Anspruch 1, dadurch gekennzeichnet, daß dann, wenn $R^2$ in der Formel (I) für $—CH_2COOR^3$ steht, $R^3$ Na, K, 1/2Ca, 1/2Mg, 1/3Al, Methyl, Ethyl, Propyl oder Butyl ist.

4. Saccharidderivat nach Anspruch 1, dadurch gekennzeichnet, daß es aus o-Aminobenzoesäureamid-N-D-fructosid, m-Aminobenzoesäureamid-N-cellobiosid, p-Aminobenzoesäureamid-N-D-xylosid, p-Aminobenzoesäureamid-N-D-mannosid oder p-Aminobenzoesäureamid-N-L-rhamnosid besteht.

5. Saccharidderivat nach Anspruch 1, dadurch gekennzeichnet, daß es aus o-Aminobenzoat-N-L-rhamnosid, Phenyl-m-aminobenzoat-N-D-mannosid, Phenyl-p-aminobenzoat-N-D-xylosid, Phenyl-p-aminobenzoat-N-cellobiosid oder Phenyl-p-aminobenzoat-N-maltotriosid besteht.

6. Saccharid nach Anspruch 1, dadurch gekennzeichnet, daß es aus Benzyl-m-aminobenzoat-N-L-fucosid, Benzyl-m-aminobenzoat-N-lactosid, Benzyl-p-aminobenzoat-N-D-deoxyribosid, Benzyl-p-aminobenzoat-N-D-glucosid oder Benzyl-p-aminobenzoat-N-D-fructosid besteht.

7. Saccharid nach Anspruch 1, dadurch gekennzeichnet, daß es aus Cyclohexyl-o-aminobenzoat-N-D-deoxyribosid, Cyclohexyl-m-aminobenzoat-N-D-mannosid, Cyclohexyl-p-aminobenzoat-N-D-xylosid, Cyclohexyl-p-aminobenzoat-N-cellobiosid, Cyclohexyl-p-aminobenzoat-N-maltotriosid, Hexahydrobenzyl-m-aminobenzoat-N-lactosid, Hexahydrobenzyl-m-aminobenzoat-N-L-fucosid, Hexahydrobenzyl-p-aminobenzoat-N-D-ribosid, Hexahydrobenzyl-p-aminobenzoat-N-D-glucosid oder Hexahydrobenzyl-p-aminobenzoat-N-L-rhamnosid besteht.

8. Saccharid nach Anspruch 1, dadurch gekennzeichnet, daß es aus Methyl-p-aminophenylacetat-N-L-rhamnosid, p-Aminophenylessigsäure-N-L-rhamnosid, Natrium-p-aminophenylacetat-N-L-rhamnosid oder Kalium-p-aminophenylacetat-N-L-rhamnosid besteht.

9. Verfahren zur Herstellung eines Saccharidderivats gemäß Anspruch 1, dadurch gekennzeichnet, daß eine Verbindung der Formel (II)

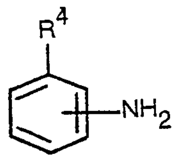

(II)

worin $R^4$ für 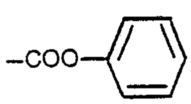 , 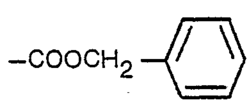 , 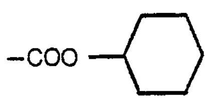 ,

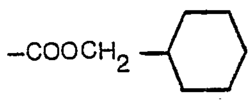 , $-CONH_2$ or $-CH_2COOR^5$

steht und $R^5$ einen Wasserstoff oder eine niedere Alkylgruppe bedeutet, mit einem Mono-, Di- oder Trisaccharid, einem Aminosaccharid, einer D-Glucuronsäure oder L-Gulonsäure umgesetzt wird und gegebenenfalls eine erhaltene Verbindung, in der $R^4$ für $—CH_2COOH$ steht, in eine Verbindung umgewandelt wird, in der $R^4$ $—CH_2COOR^6$ bedeutet, wobei $R^6$ 1 Äquivalent eines pharmazeutisch verträglichen Metalls ist.

10. Pharmazeutische Mittel aus einem Saccharidderivat gemäß einem der Ansprüche 1 bis 8 oder aus einer Verbindung, die nach einem Verfahren gemäß Anspruch 9 hergestellt worden ist, als Wirkstoff, zusammen mit einem für pharmazeutische Zwecke verträglichen Träger oder Verdünnungsmittel.

FIG. 1

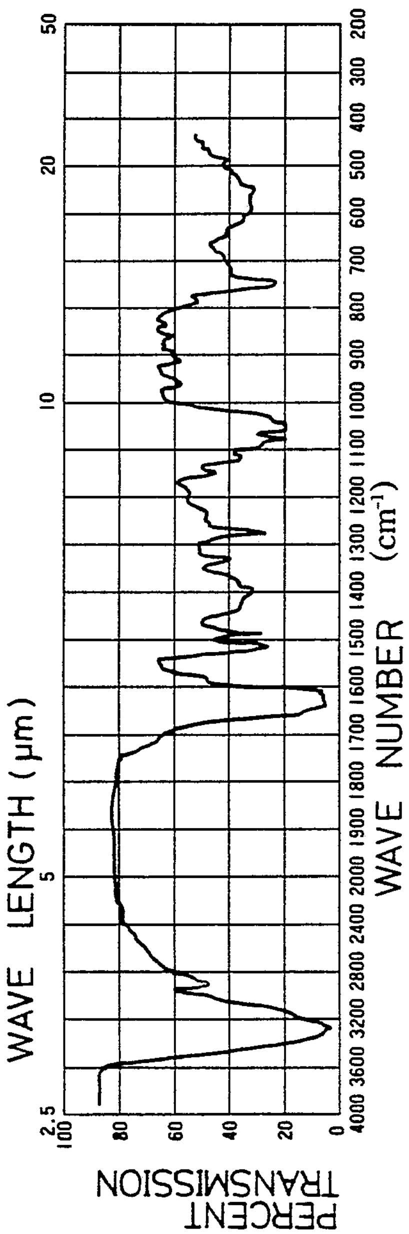
WAVE LENGTH (μm)
2.5
5
10
20
50
100
80
60
40
20
0
4000 3600 3200 2800 2400 2000 1900 1800 1700 1600 1500 1400 1300 1200 1100 1000 900 800 700 600 500 400 300 200
WAVE NUMBER (cm-1)
PERCENT TRANSMISSION

FIG. 2

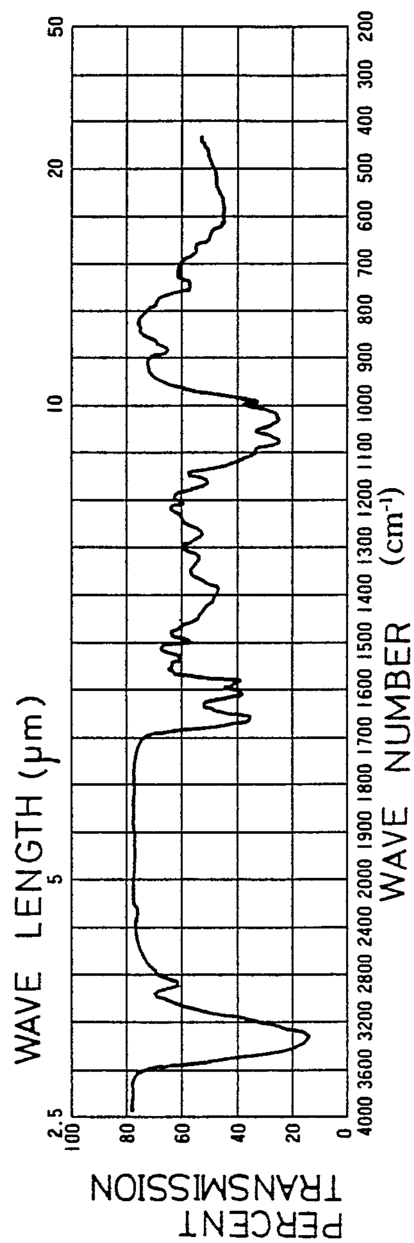
WAVE LENGTH (μm)
2.5
5
10
20
50
100
80
60
40
20
0
4000 3600 3200 2800 2400 2000 1900 1800 1700 1600 1500 1400 1300 1200 1100 1000 900 800 700 600 500 400 300 200
WAVE NUMBER (cm-1)
PERCENT TRANSMISSION

FIG. 3

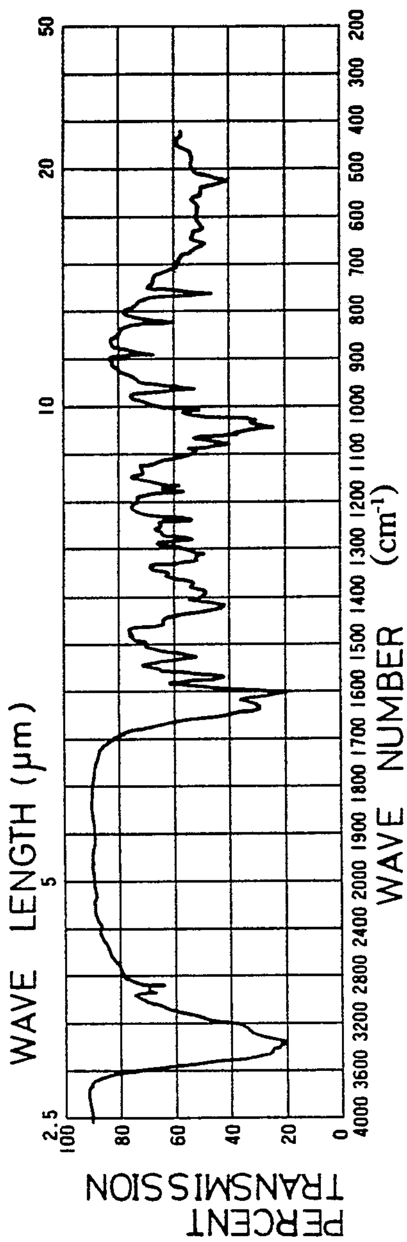
WAVE LENGTH (μm)
2.5
5
10
20
50
PERCENT TRANSMISSION
100
80
60
40
20
0
4000 3600 3200 2800 2400 2000 1900 1800 1700 1600 1500 1400 1300 1200 1100 1000 900 800 700 600 500 400 300 200
WAVE NUMBER (cm$^{-1}$)

FIG. 4

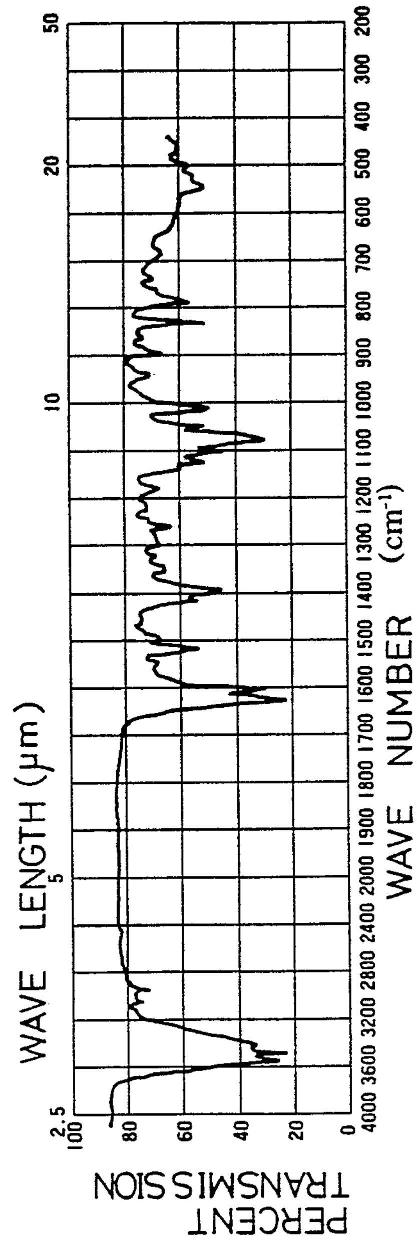
WAVE LENGTH (μm)
2.5
5
10
20
50
PERCENT TRANSMISSION
100
80
60
40
20
0
4000 3600 3200 2800 2400 2000 1900 1800 1700 1600 1500 1400 1300 1200 1100 1000 900 800 700 600 500 400 300 200
WAVE NUMBER (cm$^{-1}$)

FIG. 5

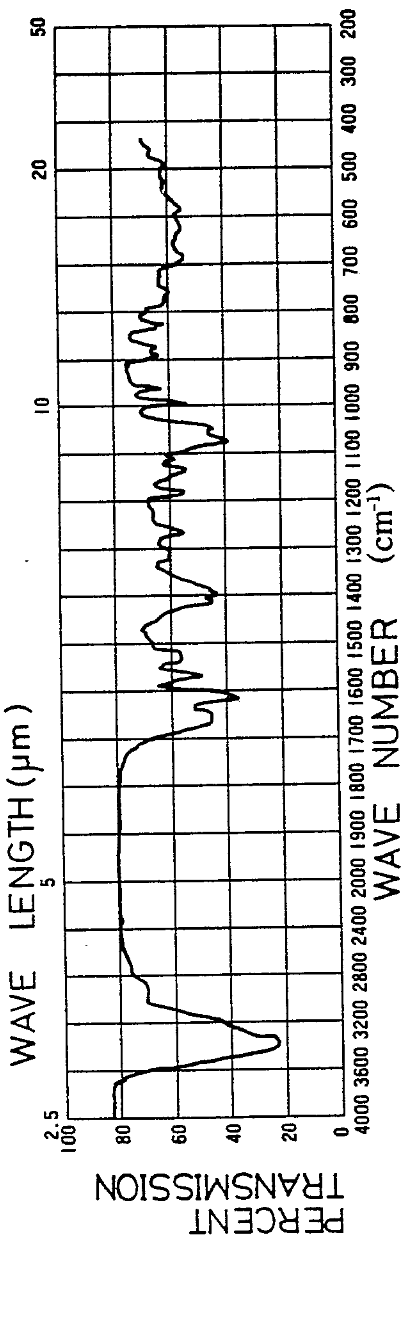
WAVE LENGTH (μm)
2.5
5
10
20
50
PERCENT TRANSMISSION
100
80
60
40
20
0
4000 3600 3200 2800 2400 2000 1900 1800 1700 1600 1500 1400 1300 1200 1100 1000 900 800 700 600 500 400 300 200
WAVE NUMBER (cm-1)

FIG. 6

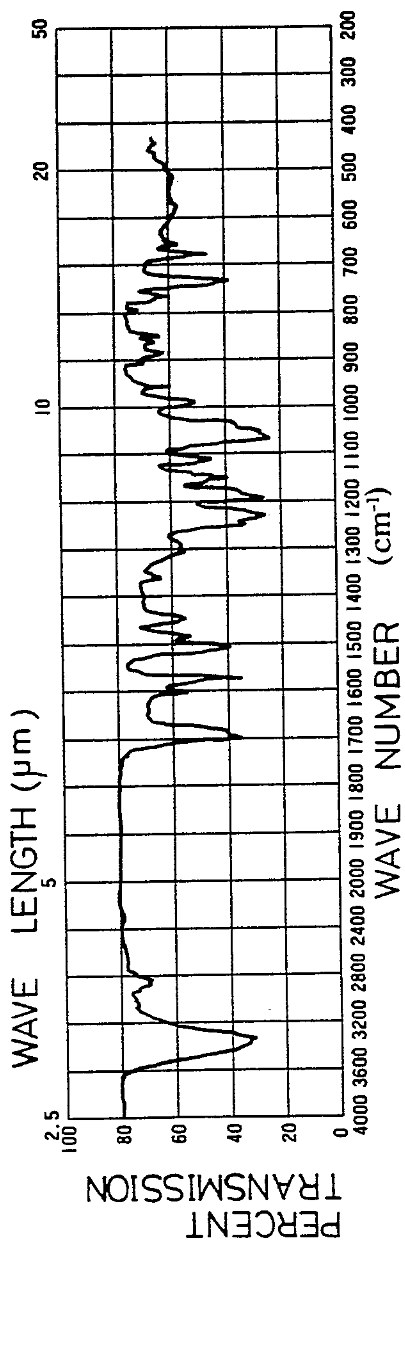
WAVE LENGTH (μm)
2.5
5
10
20
50
PERCENT TRANSMISSION
100
80
60
40
20
0
4000 3600 3200 2800 2400 2000 1900 1800 1700 1600 1500 1400 1300 1200 1100 1000 900 800 700 600 500 400 300 200
WAVE NUMBER (cm-1)

FIG. 7

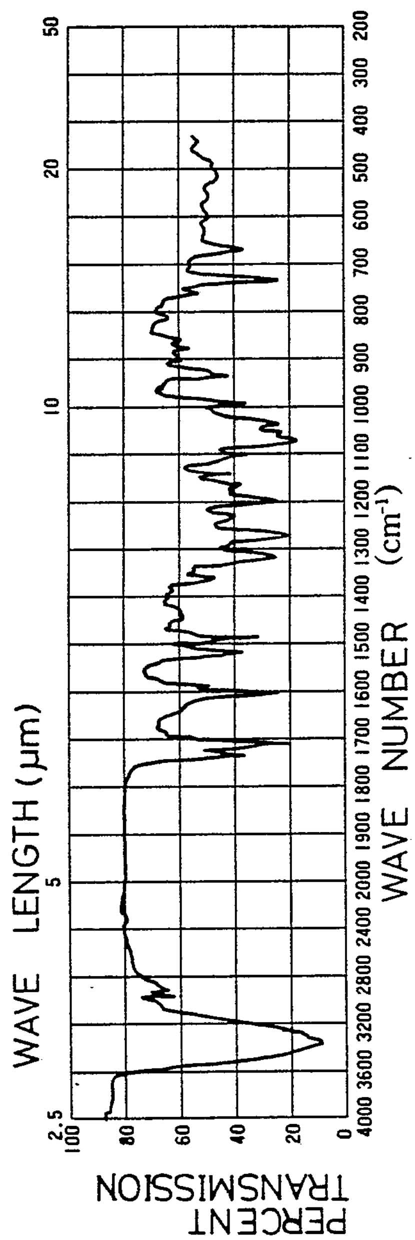
WAVE LENGTH (μm)
WAVE NUMBER (cm-1)
PERCENT TRANSMISSION

FIG. 8

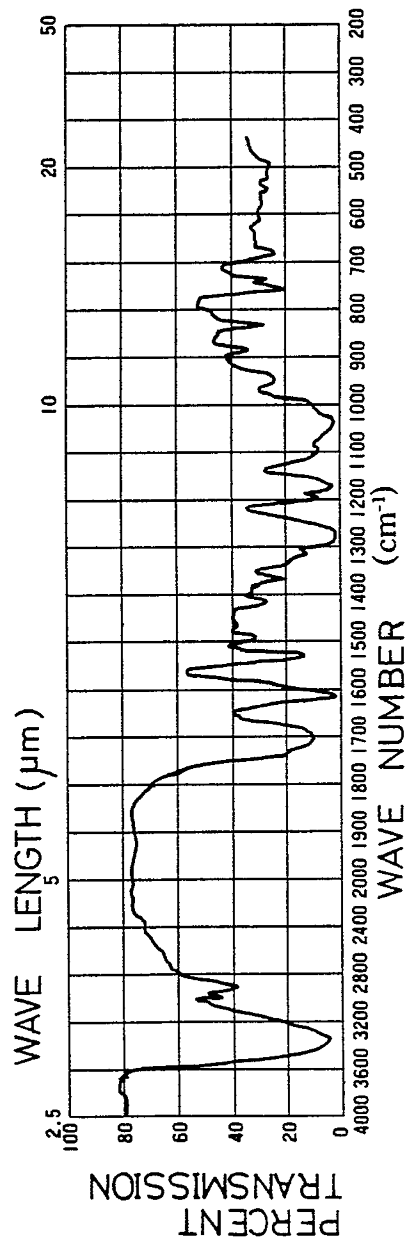
WAVE LENGTH (μm)
WAVE NUMBER (cm-1)
PERCENT TRANSMISSION

FIG. 9

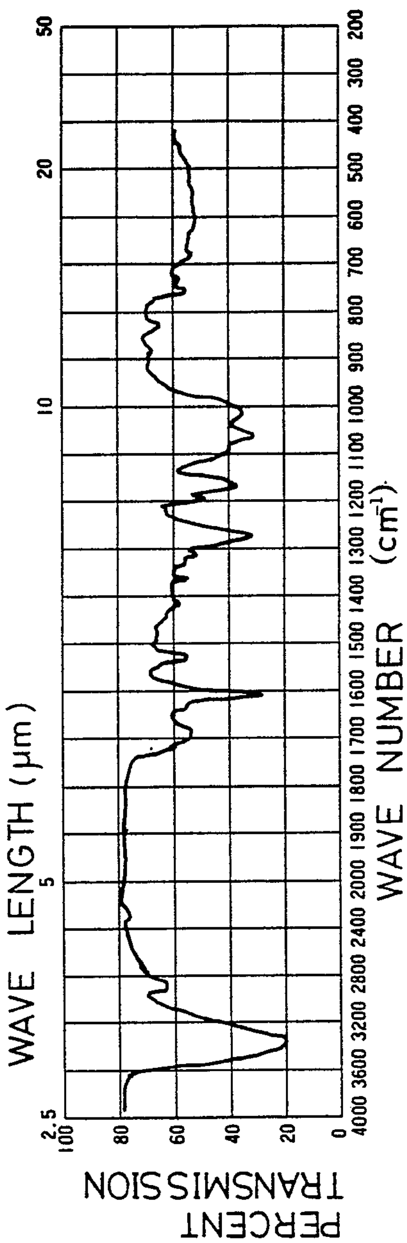
WAVE LENGTH (μm)
2.5
5
10
20
50
100
80
60
40
20
0
PERCENT TRANSMISSION
4000 3600 3200 2800 2400 2000 1900 1800 1700 1600 1500 1400 1300 1200 1100 1000 900 800 700 600 500 400 300 200
WAVE NUMBER (cm-1)

FIG. 10

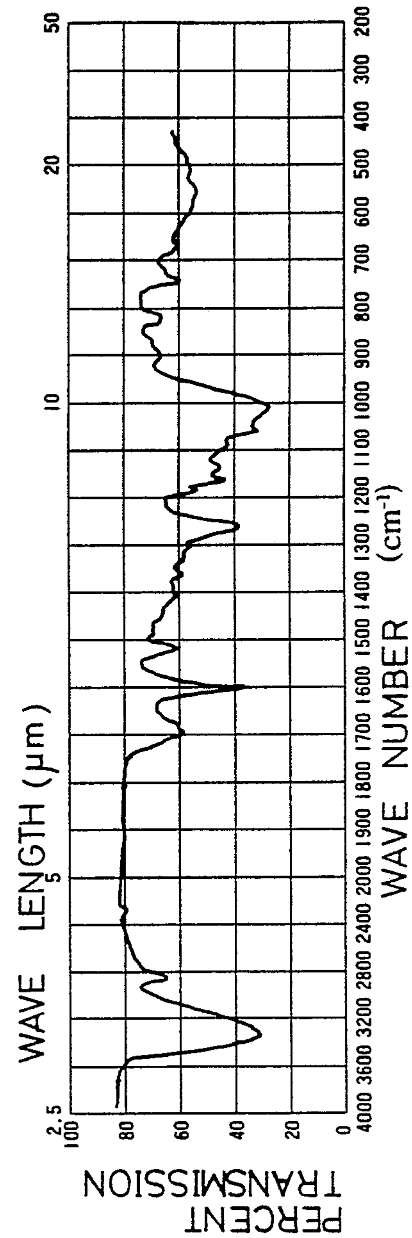
WAVE LENGTH (μm)
2.5
5
10
20
50
100
80
60
40
20
0
PERCENT TRANSMISSION
4000 3600 3200 2800 2400 2000 1900 1800 1700 1600 1500 1400 1300 1200 1100 1000 900 800 700 600 500 400 300 200
WAVE NUMBER (cm-1)

FIG. 11

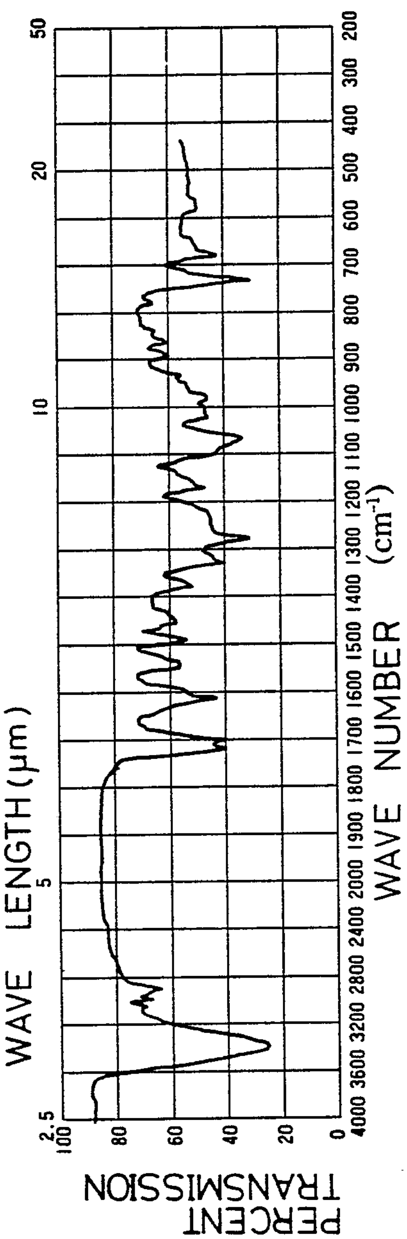
WAVE LENGTH (μm)
WAVE NUMBER (cm-1)
PERCENT TRANSMISSION

FIG. 12

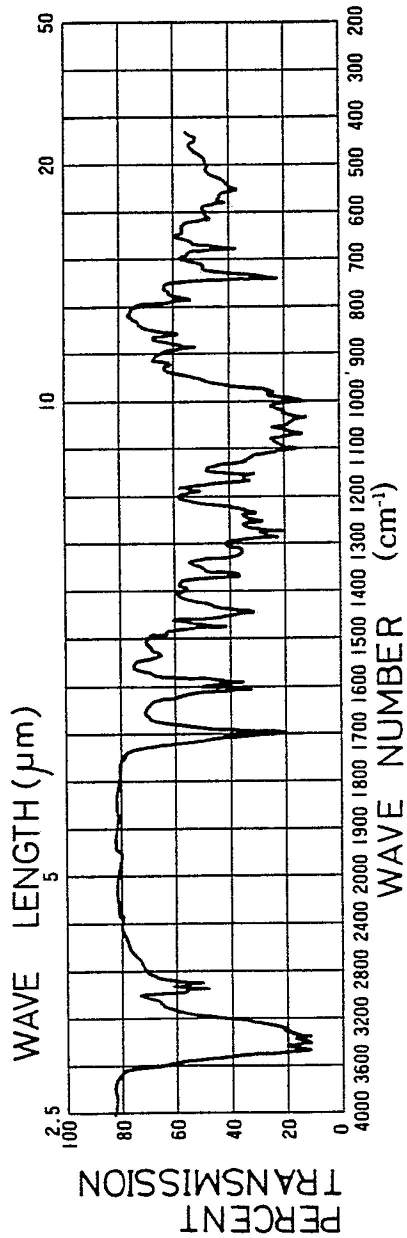
WAVE LENGTH (μm)
WAVE NUMBER (cm-1)
PERCENT TRANSMISSION

FIG. 13

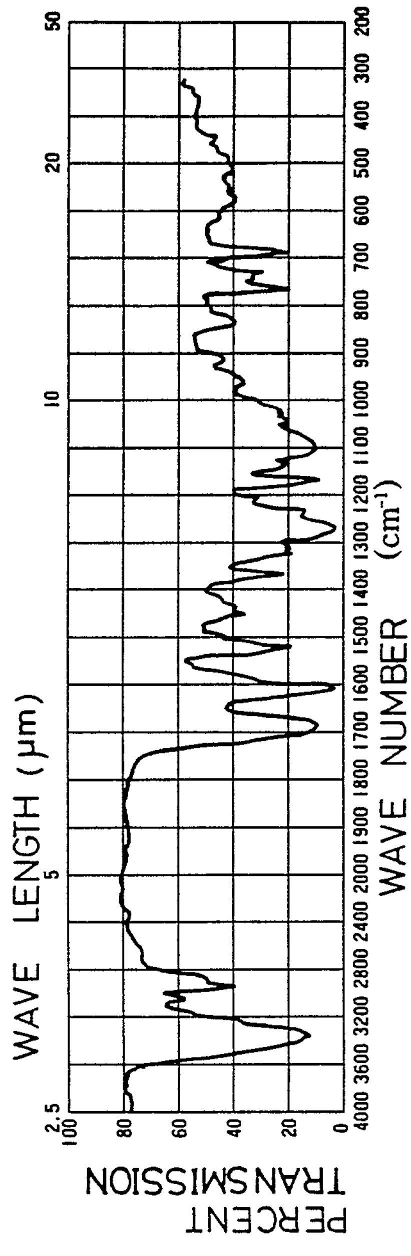

FIG. 14

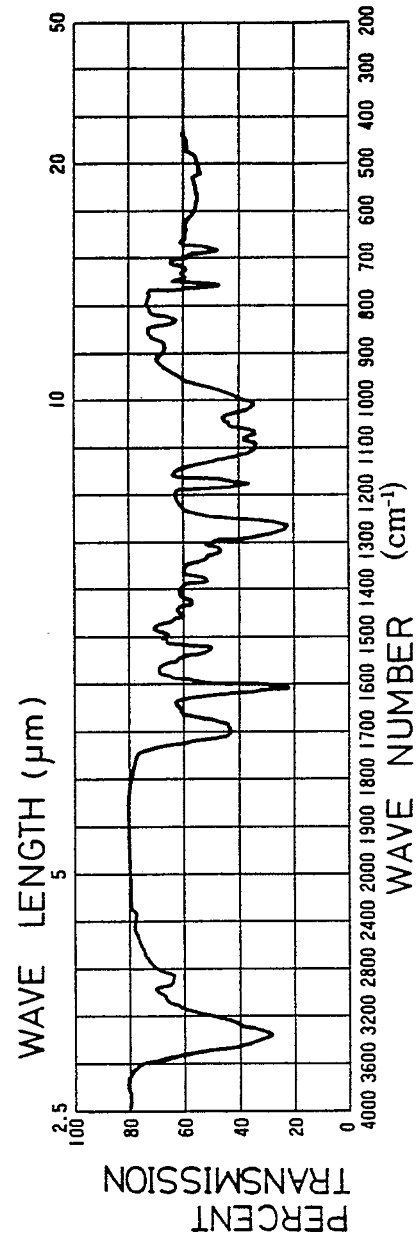

FIG. 15

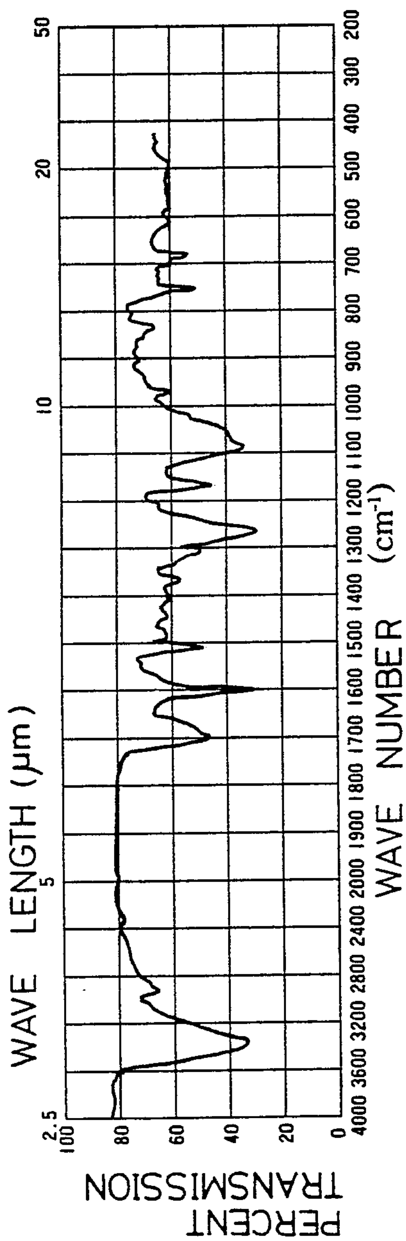

WAVE LENGTH (μm)
2.5
5
10
20
50
WAVE NUMBER (cm-1)
4000 3600 3200 2800 2400 2000 1900 1800 1700 1600 1500 1400 1300 1200 1100 1000 900 800 700 600 500 400 300 200
PERCENT TRANSMISSION
100
80
60
40
20
0


FIG. 16

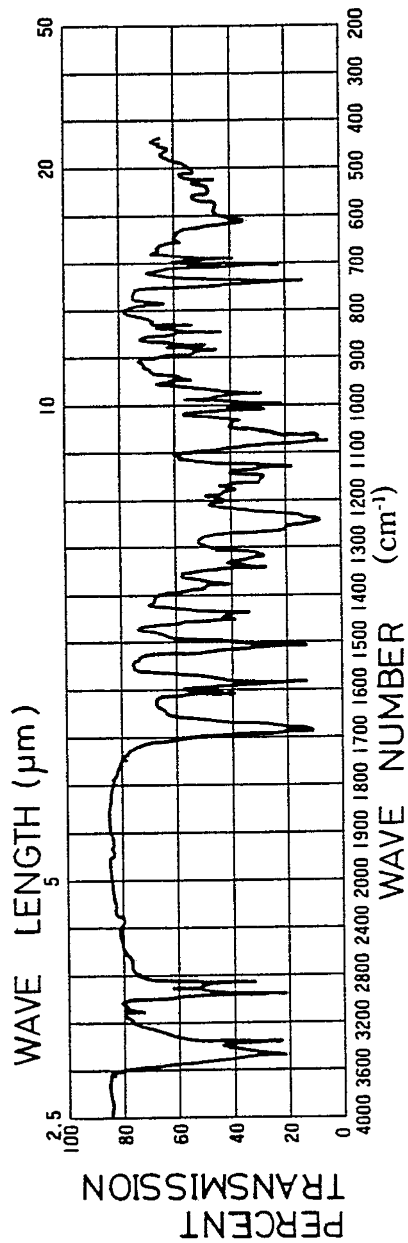

WAVE LENGTH (μm)
2.5
5
10
20
50
WAVE NUMBER (cm-1)
4000 3600 3200 2800 2400 2000 1900 1800 1700 1600 1500 1400 1300 1200 1100 1000 900 800 700 600 500 400 300 200
PERCENT TRANSMISSION
100
80
60
40
20
0

FIG. 17

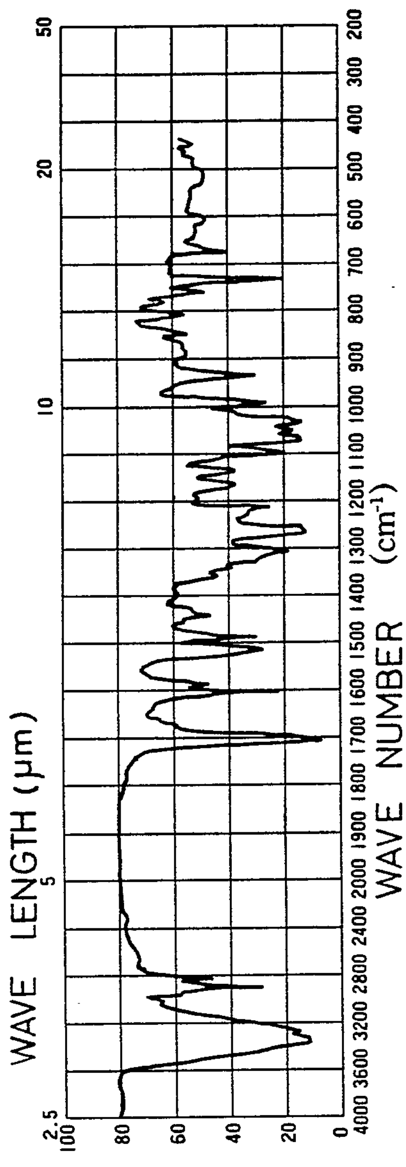
WAVE LENGTH (μm)
WAVE NUMBER (cm-1)
PERCENT TRANSMISSION

FIG. 18

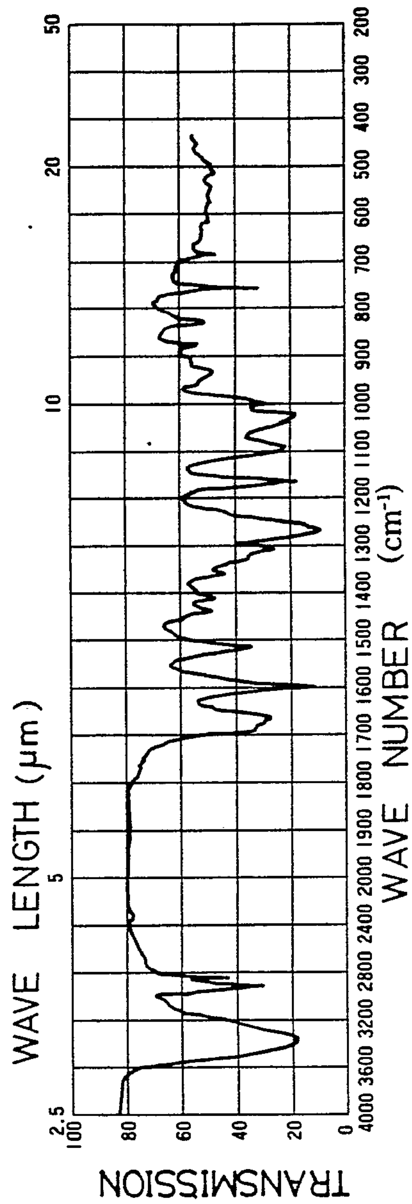
WAVE LENGTH (μm)
WAVE NUMBER (cm-1)
PERCENT TRANSMISSION

FIG. 19

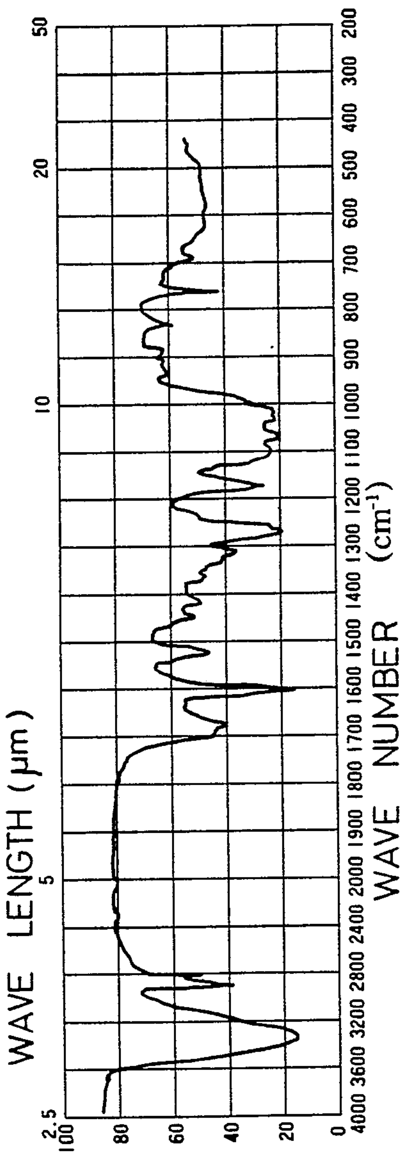
WAVE LENGTH (μm)
2.5
5
10
20
50
WAVE NUMBER $(cm^{-1})$
4000 3600 3200 2800 2400 2000 1900 1800 1700 1600 1500 1400 1300 1200 1100 1000 900 800 700 600 500 400 300 200
PERCENT TRANSMISSION
100
80
60
40
20
0

FIG. 20

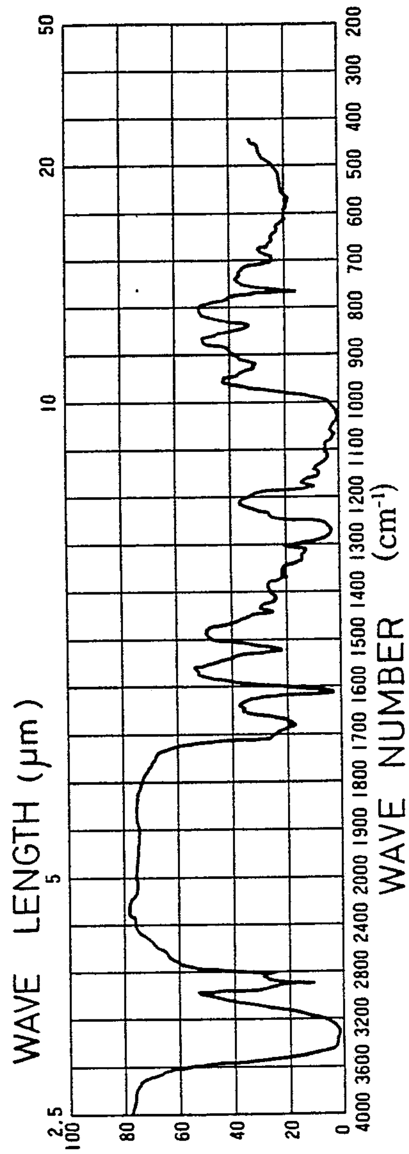
WAVE LENGTH (μm)
2.5
5
10
20
50
WAVE NUMBER $(cm^{-1})$
4000 3600 3200 2800 2400 2000 1900 1800 1700 1600 1500 1400 1300 1200 1100 1000 900 800 700 600 500 400 300 200
PERCENT TRANSMISSION
100
80
60
40
20
0

FIG. 21

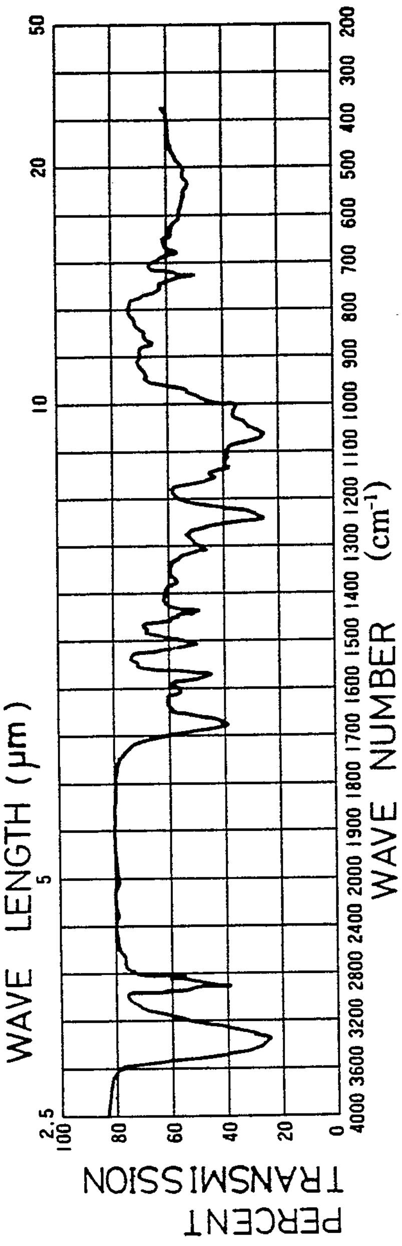
WAVE LENGTH (μm)
WAVE NUMBER (cm⁻¹)
PERCENT TRANSMISSION

FIG. 22

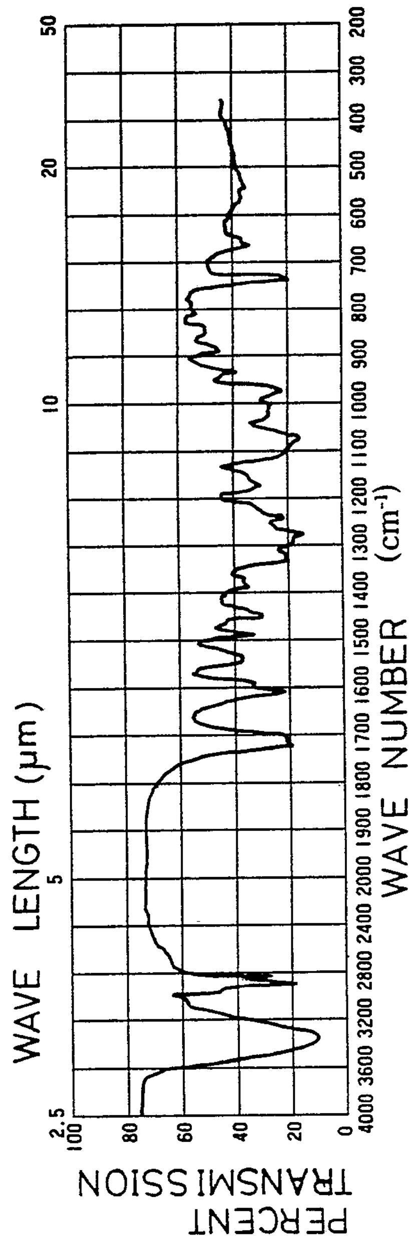
WAVE LENGTH (μm)
WAVE NUMBER (cm⁻¹)
PERCENT TRANSMISSION

FIG. 23

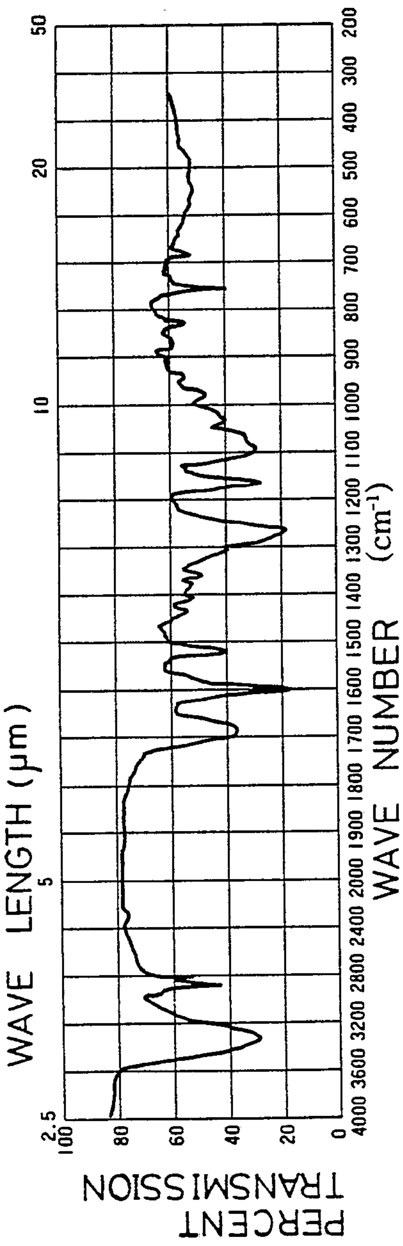

WAVE LENGTH (μm)
2.5
5
10
20
50
100
80
60
40
20
0
PERCENT TRANSMISSION
4000 3600 3200 2800 2400 2000 1900 1800 1700 1600 1500 1400 1300 1200 1100 1000 900 800 700 600 500 400 300 200
WAVE NUMBER (cm⁻¹)


FIG. 24

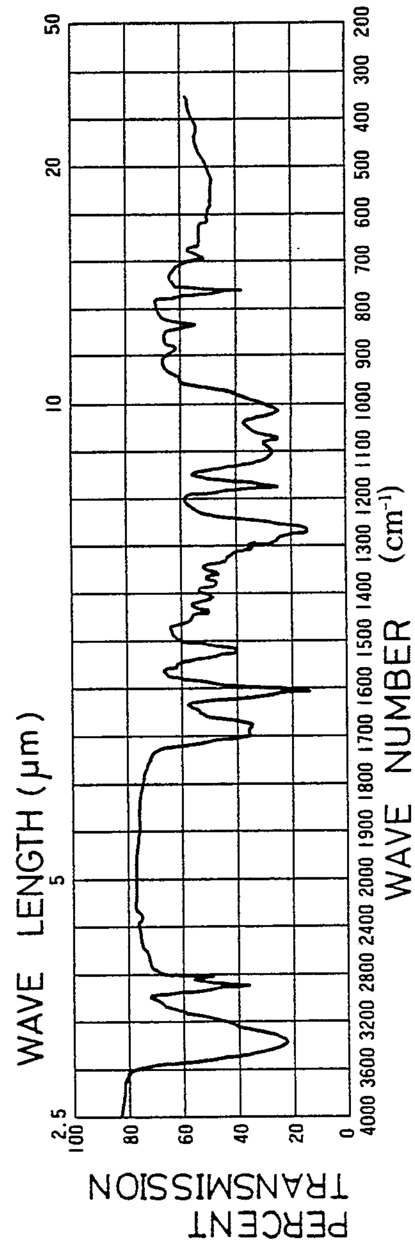

WAVE LENGTH (μm)
2.5
5
10
20
50
100
80
60
40
20
0
PERCENT TRANSMISSION
4000 3600 3200 2800 2400 2000 1900 1800 1700 1600 1500 1400 1300 1200 1100 1000 900 800 700 600 500 400 300 200
WAVE NUMBER (cm⁻¹)

FIG. 25

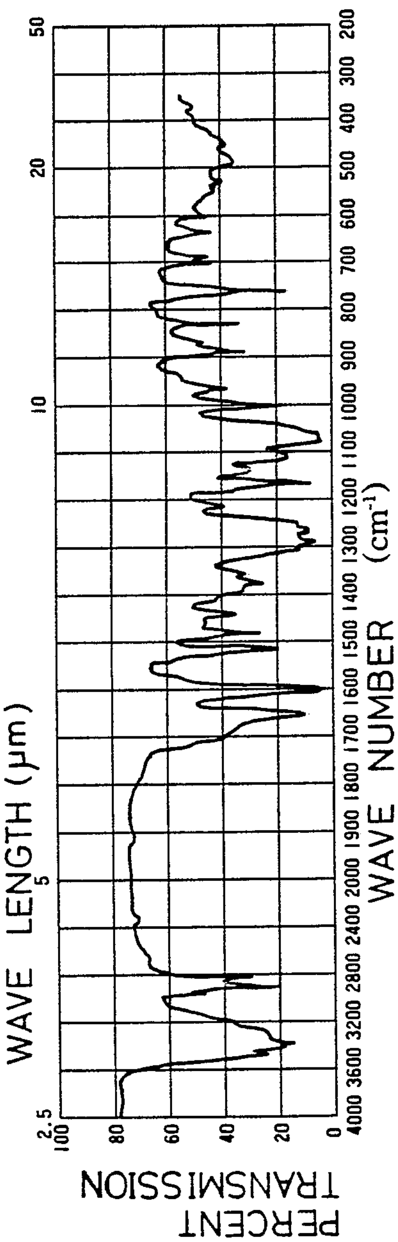
WAVE LENGTH (μm)
2.5
5
10
20
50
PERCENT TRANSMISSION
100
80
60
40
20
0
4000 3600 3200 2800 2400 2000 1900 1800 1700 1600 1500 1400 1300 1200 1100 1000 900 800 700 600 500 400 300 200
WAVE NUMBER (cm-1)

FIG. 26

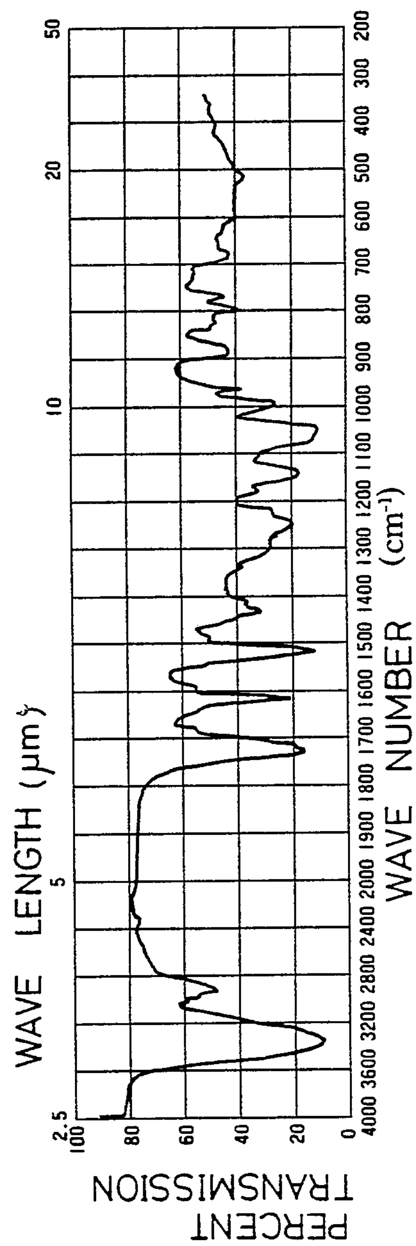
WAVE LENGTH (μm)
2.5
5
10
20
50
PERCENT TRANSMISSION
100
80
60
40
20
0
4000 3600 3200 2800 2400 2000 1900 1800 1700 1600 1500 1400 1300 1200 1100 1000 900 800 700 600 500 400 300 200
WAVE NUMBER (cm-1)

FIG. 27

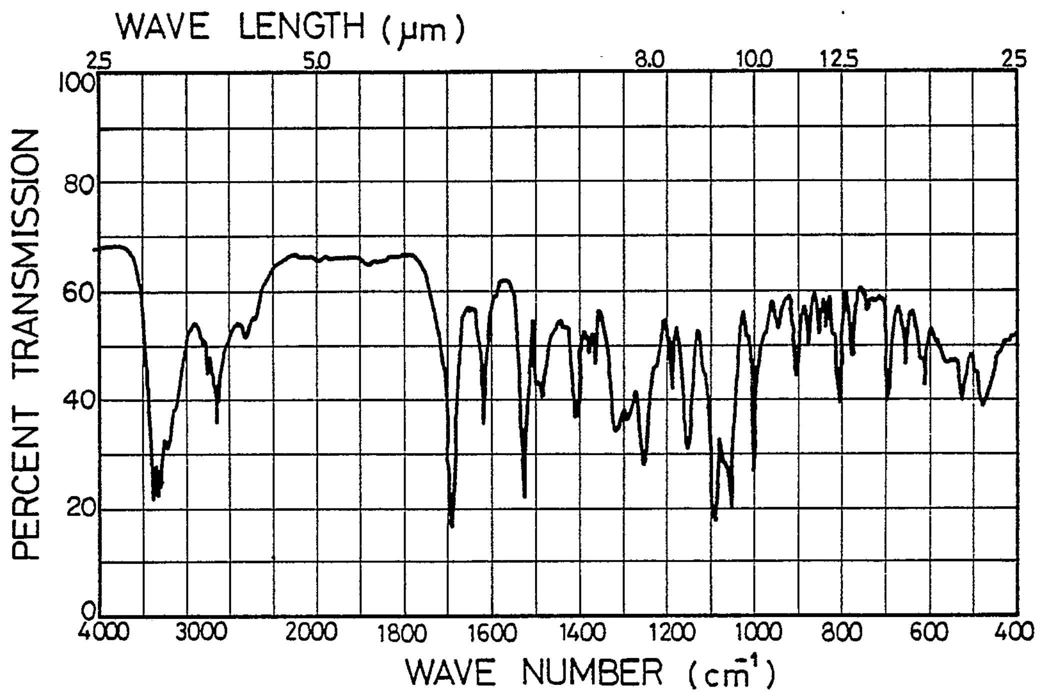
WAVE LENGTH (μm)
2.5
5.0
8.0
10.0
12.5
25
PERCENT TRANSMISSION
100
80
60
40
20
0
4000
3000
2000
1800
1600
1400
1200
1000
800
600
400
WAVE NUMBER (cm⁻¹)

FIG. 28

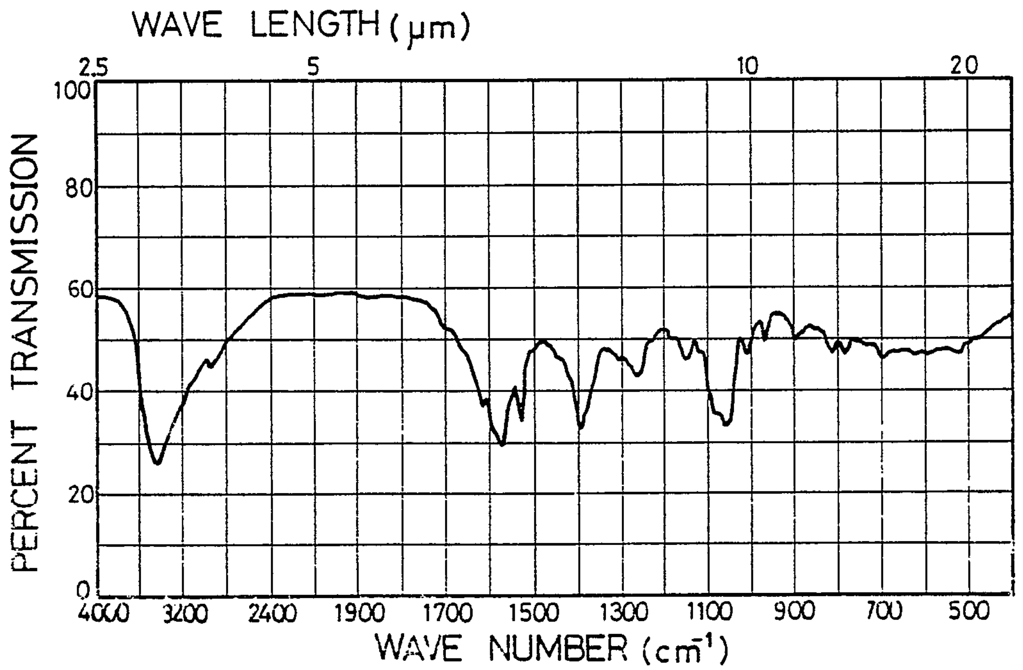
WAVE LENGTH (μm)
2.5
5
10
20
PERCENT TRANSMISSION
100
80
60
40
20
0
4000
3200
2400
1900
1700
1500
1300
1100
900
700
500
WAVE NUMBER (cm⁻¹)

# FIG. 29

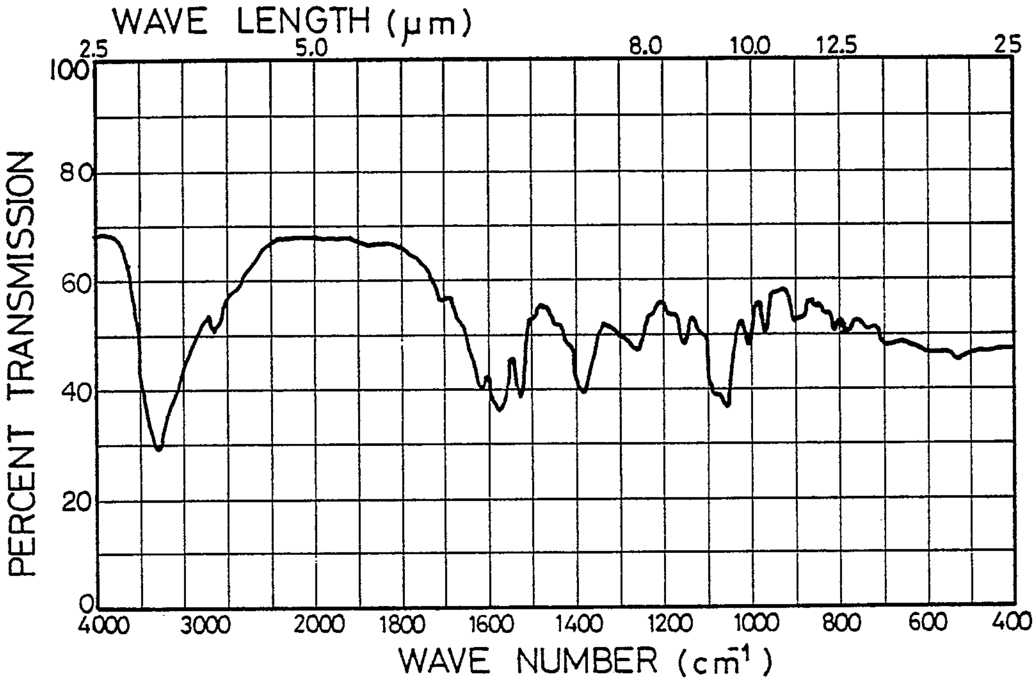

WAVE LENGTH (μm)
2.5
5.0
8.0
10.0
12.5
25
PERCENT TRANSMISSION
100
80
60
40
20
0
4000
3000
2000
1800
1600
1400
1200
1000
800
600
400
WAVE NUMBER (cm⁻¹)